# FASCICULE DE BREVET EUROPEEN

(11) **EP 2 321 323 B1**
(45) Date de publication et mention de la délivrance du brevet: **29.05.2013**
(21) Numéro de dépôt: 09802516.6
(22) Date de dépôt: 29.07.2009
(51) Int. Cl.: C07D 493/18, A61K 31/357, A61P 35/00

(54) **DERIVES DIMERIQUES D'ARTEMISININE ET APPLICATION EN THERAPIE ANTICANCEREUSE**
DIMERE ARTEMISININDERIVATE UND IHRE ANWENDUNG IN DER KREBSTHERAPIE
DIMERIC DERIVATIVES OF ARTEMISININ AND APPLICATION IN ANTI-CANCER THERAPY

(30) Priorité: 29.07.2008 FR 0855201
(43) Date de publication de la demande: 18.05.2011
(73) Titulaire: Pierre Fabre Medicament, 92100 Boulogne-Billancourt (FR); Centre National de la Recherche Scientifique (CNRS), 75016 Paris (FR); UNIVERSITE PARIS-SUD 11, 91400 Orsay (FR)
(72) Inventeur: BEGUE, Jean-Pierre, F-75012 Paris (FR); BONNET-DELPON, Danièle, F-75020 Paris (FR); CROUSSE, Benoît, F-91430 Igny (FR); FOURNIAL, Anaïs, F-53800 Renaze (FR); MORDANT, Céline, F-74160 Saint Julien En Genevois (FR); FAHY, Jacques, F-81290 Labruguiere (FR)
(74) Mandataire: Regimbeau
(86) Numéro de dépôt international: PCT/EP2009/059792
(87) Numéro de publication internationale: WO 2010/012761

(56) Documents cités:
- WO-A1-2007/116135
- US-A1- 2004 072 896
- GRELLEPOIS FABIENNE ET AL: "Synthesis of New Artemisinin-Derived Dimers by Self-Cross-Metathesis Reaction" ORGANIC LETTERS, AMERICAN CHEMICAL SOCIETY, COLUMBUS, OH; US, vol. 7, no. 23, 1 janvier 2005 (2005-01-01), pages 5219-5222, XP002477156 ISSN: 1523-7060 [extrait le 2005-10-13]
- POSNER G H ET AL: "ANTIMALARIAL, ANTIPROLIFERATIVE, AND ANTITUMOR ACTIVITIES OF ARTEMISININ-DERIVED, CHEMICALLY ROBUST, TRIOXANE DIMERS" JOURNAL OF MEDICINAL CHEMISTRY, US AMERICAN CHEMICAL SOCIETY. WASHINGTON, vol. 42, no. 21, 21 octobre 1999 (1999-10-21), pages 4275-4280, XP001061756 ISSN: 0022-2623

## Description

La présente invention concerne des dérivés dimériques de la 10-trifluorométhylartémisinine reliés d'une part par un carbone en position 10 et d'autre part par un carbone en position 16, ainsi que leur utilisation dans le traitement contre le cancer.

Les propriétés cytotoxiques des dérivés de l'artémisinine ont été mises en évidence en 1992 *[*Acta Pharmacol. Sin., 13, 541-3, (1992)], conférant ainsi à ces composés une utilisation potentielle en tant qu'anticancéreux. Il est ensuite apparu que les dérivés dimériques de l'artémisinine possédaient des activités cytotoxiques parfois supérieures à celle des monomères correspondants [J. Nat. Prod., 56, 849-56, (1993), J. Nat. Prod., 60, 325-30, (1997)].

Par conséquent, de nombreux travaux visant à préparer de nouveaux dimères d'artémisinine ont été entrepris par plusieurs équipes de recherche à travers le monde. La plupart de ces dimères sont des dimères en C-10, c'est-à-dire reliés par leur carbone en position 10, de dérivés éthers de la dihydroartémisinine *[*Bioorg. Med. Chem., 5, 1257-65, (1997)] ou d'analogues non cétaliques plus stables métaboliquement, où l'atome d'oxygène exocyclique de la fonction cétal a été remplacé par un groupement CH₂ (groupement X sur le schéma ci-dessous) *[*J. Med. Chem., 42, 4275-80, (1999)]. Des dimères en C-16 ont également été décrits [J. Med. Chem., 44, 4688-95, (2001)].

Ces différentes familles sont schématisées ci-dessous :

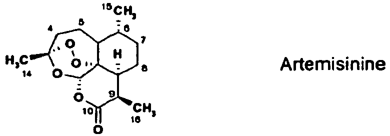

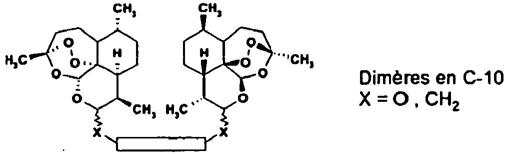

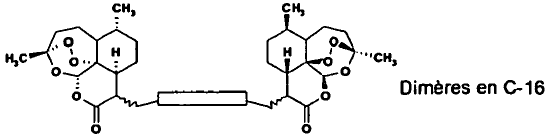

L'artémisinine et ses dérivés tels que l'arthéméther ou l'artésunate de sodium, pour les plus connus, sont largement utilisés dans le traitement du paludisme. Toutefois, la principale limitation de ces dérivés réside dans la faible biodisponibilité du noyau artémisinine, dont la fonction cétal est rapidement hydrolysée dans l'organisme, conduisant alors à des métabolites inactifs [J. Med. Chem., 47, 2945-64, (2004)].

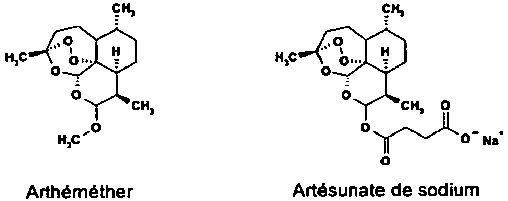

Dans le cadre de la recherche de nouveaux dérivés stables d'artémisinine pour le traitement du paludisme, des études menées au Laboratoire BioCIS de la Faculté de Pharmacie de Chatenay-Malabry ont abouti à la synthèse de dérivés 10-trifluorométhylés de l'artémisinine (WO 03/035651). L'introduction d'un groupement trifluorométhyle stabilise la fonction cétalique, ce qui a pour conséquence d'augmenter de façon très significative la stabilité de ces composés et de prolonger leur durée d'action, notamment lors d'administration des composés par voie orale.

Une revue récente détaille avec précision les avantages des dérivés trifluorométhylés de l'artémisinine sur les plans chimiques et pharmacologiques [J.-P. Bégué, D. Bonnet-Delpon, ChemMedChem, 2, 608-24, (2007)].

Une première série de dimères de 10-trifluorométhylartémisinine liés par leurs atomes de carbone en position C16 a été développée avec des profils d'activité anti-proliférative satisfaisants (demande FR 07/60266).

La présente invention concerne plus spécifiquement des dimères de 10-trifluorométhylartémisinine liés en position C10 sur un noyau et C16 sur l'autre noyau, composés qui présentent une meilleure cytotoxicité que celle des dimères liés par leurs atomes de carbone en position C16 tels que décrits dans FR 07/60266.

La présente invention concerne également des dimères de 10-trifluorométhylartémisinine dans lesquels un seul des ponts endoperoxydes est réduit en pont éther, soit du côté C10, soit du côté C16. En effet les dimères de type « mono-endoperoxyde » conservent des propriétés cytotoxiques remarquables, et plus particulièrement les dimères dans lesquels le pont endoperoxyde côté C16 a été réduit.

La présente invention a plus particulièrement pour objet un dérivé dimérique de l'artémisinine 10-trifluorométhylée de formule (I) : 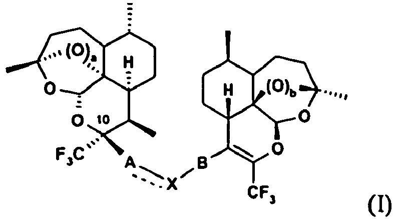 ou un sel pharmaceutiquement acceptable de celui-ci, pour lequel :
- a et b représentent, indépendamment l'un de l'autre, 1 ou 2 mais ne peuvent représenter en même temps 1,
- A représente :
   o un hétéroatome choisi parmi un atome d'azote, d'oxygène et de soufre, l'atome d'azote étant éventuellement substitué par un radical R1 choisi parmi un atome d'hydrogène, un groupement (C₁-C₆)alkyle, (C₂-C₆)alcényle, (C₂-C₆)alcynyle, (C₃-C₈)cycloalkyle, aryl-(C₁-C₆)alkyle, hétéroaryl-(C₁-C₆)alkyle, hétérocycle-(C₁-C₆)alkyle, aryle éventuellement substitué, hétéroaryle éventuellement substitué, -COR₂, -CO₂R2, - C(O)NR2R2bis, -SO₂R2,-CH₂C(O)OR2 et -CH₂C(O)NR2R2bis,
      avec R2 représentant un atome d'hydrogène ou un groupement (C₁-C₆)alkyle, (C₂-C₆)alcényle, (C₂-C₆)alcynyle, polyamino éventuellement substitué, (C₃-C₈)cycloalkyle, aryl-(C₁-C₆)alkyle, hétéroaryl-(C₁-C₆)alkyle, aryle éventuellement substitué ou hétéroaryle éventuellement substitué, et R2bis représentant un atome d'hydrogène ou un groupement (C₁-C₆)alkyle, ou
   o un hétérocycle saturé comprenant un ou plusieurs hétéroatomes choisis parmi les atomes d'oxygène, de soufre et d'azote, dont au moins un atome d'azote lié au carbone 10,
- 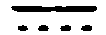 représente une liaison simple lorsque A représente un atome d'oxygène ou de soufre ou un hétérocycle, ou représente une liaison simple ou une liaison double lorsque A représente un atome d'azote, ledit atome d'azote étant substitué par un radical R1 tel que défini ci-dessus lorsque 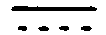 représente une liaison simple,
- B représente un groupe -CH₂-Y-, -C(=O)-Y- ou -CH(OR3)-,
   avec Y représentant O, S, N-R1 ou un hétérocycle, R1 étant tel que défini ci-dessus, et
   R3 représentant un atome d'hydrogène ou un groupement (C₁-C₆)alkyle ou aryle, et
- X représente :
   ■ lorsque 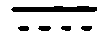 représente une liaison double :
      un groupement =C(X1)-(O-X2)_{c}- avec X1 et X2 représentant, indépendamment l'un de l'autre, un groupement (C₁-C₆)alkyle ou (C₂-C₆)alcényle et c représentant 0 ou 1, ou
   ■ lorsque 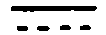 représente une liaison simple :
      o un groupement (C₁-C₆)alkyle éventuellement substitué par un ou plusieurs groupes OH; (C₂-C₆)alcényle; (C₂-C₆)alcynyle; [(C₁-C₆)alkyl]ₙ-(C₃-C₈)cycloalkyl-[(C₁-C₆)alkyle]ₚ ; [(C₁-C₆)akyl]ₙ-hétérocycle-[(C₁-C₆)alkyle]ₚ ; [(C₁-C₆)alkyl]ₙ-aryl-[(C₁-C₆)alkyle]ₚ ; [(C₁-C₆)alkyl]ₙ-hétéroaryl-[(C₁-C₆)alkyle]ₚ ; avec n et p représentant, indépendamment l'un de l'autre, 0 ou 1,
      o un groupement -CO-(CH₂)_{q}- ou -CO-(CH₂)_{q}-CO- pour lequel q représente un nombre entier égal à 1, 2, 3 ou 4, ou
      o un groupement -COᵣ-(CH₂)ₛ-Z-(CH₂)ₜ-COᵤ- pour lequel r et u représentent, indépendamment l'un de l'autre, un nombre entier égal à 0 ou 1,
         s et t représentent, indépendamment l'un de l'autre, un nombre entier égal à 0, 1, 2, 3 ou 4, et
         Z représente un groupe -S-, -S-S-, -SO-, -SO₂-, -Se-Se-, -O-P(O)(OR3)-O-, -NR1-, (C₃-C₈)-cycloalkyle, aryle ou hétéroaryle, avec R1 et R3 tels définis ci-dessus.

Les dérivés dimériques de la présente invention possèdent ainsi les avantages des monomères fluorés évoqués précédemment tout en présentant de bonnes propriétés antitumorales.

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; et
(3) les sels formés lorsqu'un proton acide présent dans le composé parent soit est remplacé par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium ; soit se coordonne avec une base organique ou inorganique. Les bases organiques acceptables comprennent la diéthanolamine, l'éthanolamine, N-méthytgtucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Le terme « halogène » désigne un fluor, un brome, un chlore ou un iode. De préférence, il s'agit d'un fluor, d'un brome ou d'un chlore, et encore de préférence d'un fluor.

Par groupement « (C₁-C₆)alkyle », on entend, au sens de la présente invention, une chaîne hydrocarbonée saturée, linéaire ou ramifiée, comportant de 1 à 6 atomes de carbone, comme par exemple un groupement méthyle, éthyle, propyle, isopropyle, butyle, *tertio-*butyle, pentyle ou encore hexyle. Selon les cas, cette chaîne sera monovalente ou bivalente. Dans le cas d'une chaîne bivalente, il s'agira avantageusement d'une chaîne linéaire de formule -(CH₂)ₙ- où n représente un nombre entier compris entre 1 et 6 de préférence égal à 1,2,3 ou 4.

Par groupement « (C₂-C₆)alcényle », on entend, au sens de la présente invention, une chaîne hydrocarbonée insaturée, linéaire ou ramifiée, comportant de 1 à 6 atomes de carbone, et comportant au moins une double liaison, et avantageusement une seule. Il s'agit notamment d'un groupe vinyle ou allyle. Selon les cas, cette chaîne sera monovalente ou bivalente. Dans le cas d'une chaîne bivalente, il s'agira avantageusement d'une chaîne linéaire telle qu'une chaîne de formule -CH₂-CH=CH-CH₂-.

Par groupement « (C₂-C₆)alcynyle », on entend, au sens de la présente invention, une chaîne hydrocarbonée insaturée, linéaire ou ramifiée, comportant de 1 à 6 atomes de carbone, et comportant au moins une triple liaison, et avantageusement une seule. Il s'agit notamment d'un groupe éthynyle ou propynyle. Selon les cas, cette chaîne sera monovalente ou bivalente. Dans le cas d'une chaîne bivalente, il s'agira avantageusement d'une chaîne linéaire telle qu'une chaîne de formule -CH₂-C≡C-.

Par groupement « polyamino », on entend, au sens de la présente invention, une chaîne hydrocarbonée linéaire comprenant de 6 à 20 atomes de carbone, et dont au moins deux de ces atomes de carbone sont remplacés par des atomes d'azote, deux atomes d'azote ne pouvant se retrouver sur des positions adjacentes. Ledit groupe polyamino pourra répondre notamment à la formule suivante :

-[(CH₂)ₐ-NH]_{d}-[(CH₂)_{b}-NH-(CH₂)_{c}-NH]ₑ-H

avec a, b et c représentant, indépendamment les uns des autres, un nombre entier compris entre 1 et 5 et d et e représentant chacun 0 ou 1

A titre d'exemple, il peut être cité une chaîne de type spermidine, c'est-à-dire de formule -(CH₂)₄-NH-(CH₂)₃-NH₂ ou bien -(CH₂)₃-NH-(CH₂)₄-NH₂, une chaîne de type spermine de formule -(CH₂)₃-NH-(CH₂)₄-NH-(CH₂)₃-NH₂ ou encore une chaîne de formule -(CH₂)₄-NH-(CH₂)₄-NH-(CH₂)₄-NH₂.

Ce groupe polyamino peut être éventuellement substituée, plus particulièrement sur les atomes d'azote, notamment par un groupe N-protecteur tel que (C₁-C₆)alkyle, (C₂-C₆)alcényle, -CO-(C₁-C₆)alkyle, -CO-(C₂-C₆)alcényle, -CO₂-(C₁-C₆)alkyle ou -CO₂-(C₂-C₆)alcényle.

Ledit groupe polyamine éventuellement substituée pourra alors répondre à la formule générale suivante :

-[(CH₂)ₐ-NA₁]_{d}-[(CH₂)_{b}-NA₂-(CH₂)_{c}-NA₃]ₑ-H,

avec a, b, c, d et e tels que définis ci-dessus et A₁, A₂ et A₃, différents ou de préférence identiques, représentant un atome d'hydrogène ou un groupe N-protecteur tel que (C₁-C₆)alkyle, (C₂-C₆)alcényle, -CO-(C₁-C₆)alkyle, -CO-(C₂-C₆)alcényle, -CO₂-(C₁-C₆)alkyle ou -CO₂-(C₂-C₆)alcényle.

Par « groupe protecteur », on entend désigner, au sens de la présente invention, un groupe qui bloque sélectivement un site réactif dans un composé multifonctionnel de telle sorte qu'une réaction chimique peut être effectuée sélectivement au niveau d'un autre site réactif non protégé dans la signification classiquement associée à celui-ci en chimie de synthèse.

Par « groupe N-protecteur », on entend, au sens de la présente invention, tout substituant qui protège le groupe NH ou NH₂ contre les réactions indésirables tels que les groupes N-protecteur décrits dans Greene, « Protective Groups In Organic synthesis », (John Wiley & Sons, New York (1981)) et Harrison et al. « Compendium of Synthetic Organic Methods », Vols. 1 à 8 (J. Wiley & sons, 1971 à 1996). Les groupes N-protecteur comprennent les carbamates (tel que -CO₂-(C₁-C₆)alkyle ou -CO₂-(C₂-C₆)alcényle), les amides (tel que -CO-(C₁-C₆)alkyle, -CO-(C₂-C₆)alcényle), les dérivés N-alkylés ou N-alcénylés, les dérivés amino acétale, les dérivés N-benzylés, les dérivés imine, les dérivés énamine et les dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (Boc), le benzyloxycarbonyle (Cbz), le p-méthoxybenzyloxycarbonyle, le p-nitrobenzyl-oxycarbonyle, le trichloroéthoxycarbonyle (TROC), l'allyloxycarbonyle (Alloc), le 9-fluorénylméthyloxycarbonyle (Fmoc), le trifluoro-acétyle, les carbamates de benzyle (substitués ou non) et similaires. Il pourra s'agir en particulier d'un groupement Boc.

Par groupement « (C₃-C₈)-cycloalkyle », on entend, au sens de la présente invention, un groupement cyclique hydrocarboné saturé comportant de 3 à 8, de préférence 5 ou 6, atomes de carbone, comme par exemple un groupement cyclopropyle, cyclohexyle, cyclopentyle, etc. Selon les cas, ce cycle sera monovalent ou bivalent.

Par groupement «aryle », on entend, au sens de la présente invention, un groupement aromatique, comportant de préférence de 5 à 10 atomes de carbone et comprenant un ou plusieurs cycles accolés, de préférence 1 ou 2 et encore de préférence un seul cycle, comme par exemple un groupement phényle ou naphtyle. Avantageusement, il s'agit du phényle. Selon les cas, ce groupe sera monovalent ou bivalent.

Ce groupement aryle peut être éventuellement substitué, notamment par un ou plusieurs groupements choisis parmi un atome d'halogène et un groupement (C₁-C₆)-alkyle tel que défini précédemment, -OR, -NRR' et -SO₂R avec R et R' désignant un atome d'hydrogène ou un groupement (C₁-C₆)-alkyle tel que défini précédemment.

Par groupement « noyau hétéroaryle », on entend, au sens de la présente invention, tout groupe aryle tel que défini ci-dessus dans lequel un ou plusieurs atomes de carbone ont été remplacés par un ou plusieurs hétéroatomes avantageusement 1 à 4 et, encore plus avantageusement 1 à 3, tels que par exemple des atomes de soufre, azote ou oxygène. Des exemples de groupes hétéroaryle sont les groupes furyle, thiényle, pyrrolyle, pyridinyle, pyrimidinyle, pyrazolyle, imidazolyle, triazolyle, tétrazolyle ou encore indyle. Avantageusement, il s'agit d'un groupe pyridinyle ou triazolyle tel qu'un 1,2,3-triazolyle. Selon les cas, ce groupe sera monovalent ou bivalent.

Ce groupe hétéroaryle peut être éventuellement substitué, notamment par un ou plusieurs groupements choisis parmi un atome d'halogène et un groupement (C₁-C₆)-alkyle tel que défini précédemment, -OR, -NRR' et -SO₂R avec R et R' désignant un atome d'hydrogène ou un groupement (C₁-C₆)-alkyle tel que défini précédemment.

Par « hétérocycle », on entend, au sens de la présente invention, un composé hydrocarboné cyclique saturé, insaturé ou aromatique, sauf mention contraire, comprenant un ou plusieurs cycles accolés, de préférence 1 ou 2 et encore de préférence un seul cycle, et comprenant 5 à 10 atomes cycliques, dont un ou plusieurs atomes de carbone cycliques ont été remplacés par un ou plusieurs hétéroatomes, avantageusement 1 à 4 et, encore plus avantageusement 1 à 3, tels que par exemple des atomes de soufre, azote ou oxygène. Il peut s'agir en particulier d'un groupement morpholinyle, pipérazinyle, pipéridinyle, furyle, thiényle, pyrrolyle, tétrazolyle ou encore triazolyle. Avantageusement, il s'agit d'un groupe pipérazinyle ou triazolyle tel qu'un 1,2,3-triazolyle. Selon les cas, ce groupe sera monovalent ou bivalent.

Dans le cas où l'hétérocycle est saturé, il s'agit avantageusement d'une pipéridine, d'une morpholine ou d'une pipérazine.

Par groupement « aryl-(C₁-C₆)alkyle », on entend, au sens de la présente invention, un groupement aryle tel que défini ci-dessus lié à la molécule par l'intermédiaire d'un groupement (C₁-C₆)alkyle tel que défini ci-dessus. Il s'agit en particulier d'un groupe benzyle.

Par groupement « hétéroaryl-(C₁-C₆)akyle », on entend, au sens de la présente invention, un groupement hétéroaryle tel que défini ci-dessus lié à la molécule par l'intermédiaire d'un groupement (C₁-C₆)akyle tel que défini ci-dessus. Il s'agit en particulier d'un groupe benzyle.

Par groupement « hétérocycle-(C₁-C₆)alkyle », on entend, au sens de la présente invention, un hétérocycle tel que défini ci-dessus lié à la molécule par l'intermédiaire d'un groupement (C₁-C₆)alkyle tel que défini ci-dessus. Il s'agit en particulier d'un groupe benzyle.

De manière avantageuse, a = b = 2 ou a = 2 et b = 1. De manière encore avantageuse, a = b = 2.

Selon un mode de réalisation particulier de l'invention, R1 représente un atome d'hydrogène, un groupement (C₁-C₆)alkyle, (C₂-C₆)alcényle, (C₂-C₆)alcynyle, (C₃-C₈)cycloalkyle, aryl-(C₁-C₆)alkyle, hétéroaryl-(C₁-C₆)alkyle, hétérocycle-(C₁-C₆)akyle, aryle éventuellement substitué, hétéroaryle éventuellement substitué, -COR2, -CO₂R2, ou -SO₂R2, avec R2 représentant un atome d'hydrogène ou un groupement (C₁-C₆)alkyle, (C₃-C₈)cycloalkyle, aryl-(C₁-C₆)alkyle, hétéroaryl-(C₁-C₆)alkyle, aryle éventuellement substitué ou hétéroaryle éventuellement substitué.

R1 pourra en particulier représenter un atome d'hydrogène.

Avantageusement, R1 représente un atome d'hydrogène, un groupement (C₁-C₆)alkyle, (C₂-C₆)alcényle, -CH₂C(O)OR2 ou -CH₂C(O)NR2R2bis, avec R2 tel que défini précédemment et représentant avantageusement un atome d'hydrogène ou un groupement, polyamino éventuellement substitué, et R2bis tel que défini précédemment et représentant avantageusement un atome d'hydrogène.

Comme indiqué précédemment, B représente un groupement -CH₂Y-, -C(=O)-Y-ou -CH(OR3)-, Y et R3 étant défini ci-dessus. Dans le cas des groupements -CH₂Y- et - C(=O)-Y-, l'atome de carbone de ces deux groupements est lié au noyau d'artémisinine tandis que le groupement Y est lié au groupement X dans la formule générale (I).

Lorsque Y représente un hétérocycle, celui-ci est lié au groupement CH₂ ou C(=O) du radical B, de préférence, par un atome d'azote.

De manière avantageuse, B représente un groupement -CH₂Y-, -C(=O)-Y- ou - CH(OR3)-, avec Y représentant O, NR1 ou un hétérocycle, R1 et R3 étant tels que définis ci-dessus et de préférence représentant chacun un atome d'hydrogène.

De préférence, B est choisi parmi les groupes -CH₂O-, -CH₂NR1-, -C(=O)NR1-, - CH(OR3)- et --CH₂-(hétérocycle)-, avec R1 et R3 tels que défini ci-dessus, R3 représentant avantageusement un atome d'hydrogène et R1 représentant avantageusement un atome d'hydrogène, un groupe (C₁-C₆)alkyle, (C₂-C₆)alcényle, -CH₂C(O)OR2 ou - CH₂C(O)NR2R2bis avec R2 et R2bis tels que définis ci-dessus, R2 représentant de préférence un atome d'hydrogène ou une chaîne polyamine éventuellement substituée et R2bis représentant de préférence un atome d'hydrogène. Dans ce cas, R1 et R3 pourront plus particulièrement représenter chacun un atome d'hydrogène.

L'hétérocycle sera en particulier, dans ce cas, un hétérocycle comportant un seul cycle de 5 ou 6 chaînons. De préférence, il comportera un ou plusieurs, de préférence 1 à 4, atomes d'azote, comme un groupement pipérazinyle ou triazolyle tel que 1,2,3-triazolyle.

B pourra représenter en particulier un groupement -CH₂O-, -CH₂NH-, -CH(OH)-, - C(=O)NH-, 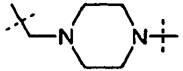 ou 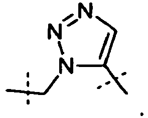 B pourra représenter également un groupement -CH₂NCH₃-, -CH₂N(CH₂CH=CH₂)-, -CH₂N(CH₂COOH)-, -CH₂N(CH₂C(O)NH(CH₂)₃NH(CH₂)₄NH(CH₂)₃NH₂)-, ou encore

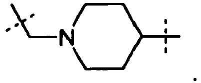

Avantageusement, B représente un groupement -CH₂-Y- avec Y tel que défini ci-dessus et de préférence représentant O, NR1 ou un hétérocycle, R1 étant tel que défini précédemment.

De manière avantageuse, A représente un atome d'oxygène ou d'azote. Lorsque A est lié à X par une liaison simple et qu'il représente un atome d'azote, l'atome d'azote sera de préférence substitué par un atome d'hydrogène.

Avantageusement, A représentera un atome d'oxygène.

X pourra représenter en particulier, de préférence lorsque 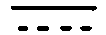 représente une liaison simple :
o un groupement (C₁-C₆)alkyle, (C₂-C₆)alcényle, (C₂-C₆)alcynyle, [(C₁-C₆)alkyl]ₙ-(C3-C₈)cycloalkyl-[(C₁-C₆)alkyle]ₚ, [(C₁-C₆)akyl]ₙ-hétérocycle-[(C₁-C₆)akyle]ₚ, [(C₁-C₆)alkyl]ₙ-aryl-[(C₁-C₆)alkyle]ₚ, [(C₁-C₆)alkyl]ₙ-hétéroaryl-[(C₁-C₆)alkyle]ₚ, avec n et p représentant, indépendamment l'un de l'autre, 0 ou 1,
o un groupement -CO-(CH₂)_{q}- ou -CO-(CH₂)_{q}-CO- pour lequel q représente un nombre entier égal à 1, 2, 3 ou 4, ou
o un groupement -COᵣ-(CH₂)ₛ-Z-(CH₂)ₜ-COᵤ- pour lequel r et u représentent, indépendamment l'un de l'autre, un nombre entier égal à 0 ou 1, s et t représentent, indépendamment l'un de l'autre, un nombre entier égal à 0, 1, 2, 3 ou 4, et
   Z représente un groupe -S-, -S-S-, -SO-, -SO₂-, -Se-Se-, -O-P(O)(OR3)-O-, -NR1-, (C₃-C₈)-cycloalkyle, aryle ou hétéroaryle, avec R1 et R3 tels définis ci-dessus.

Selon un premier mode de réalisation particulier, 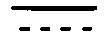 représente une liaison double et X représente un groupement (C₁-C₆)alkyle, (C₂-C₆)alcényle, (C₂-C₆)alcynyle, (C₃-C₈)cycloalkyle, ou-X1-O-X2- avec X1 et X2 tels que définis ci-dessus. En particulier, X pourra représenter un groupement (C₁-C₆)alkyle, (C₂-C₆)alcényle, (C₂-C₆)alcynyle ou (C₃-C₈)cycloalkyle, et de préférence (C₁-C₆)alkyle.

Selon un second mode de réalisation particulier, 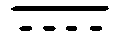 représente une liaison simple et X représente :
o un groupement (C₁-C₆)alkyle éventuellement substitué par un ou plusieurs groupes OH ; (C₂-C₆)alcényle ; (C₂-C₆)alcynyle ; [(C₁-C₆)alkyl]ₙ-(C₃-C₈)cycloalkyl-[(C₁-C₆)alkyle]ₚ; [(C₁-C₆)alkyl]ₙ-hétérocycle-[(C₁-C₆)alkyle]ₚ ; [(C₁-C₆)alkyl]ₙ-aryl-[(C₁-C₆)alkyle]ₚ ; [(C₁-C₆)alkyl]ₙ-hétéroaryl-[(C₁-C₆)alkyle]ₚ ; avec n et p représentant, indépendamment l'un de l'autre, 0 ou 1, ou
o un groupement -CO-(CH₂)_{q}- ou -CO-(CH₂)_{q}-CO- pour lequel q représente un nombre entier égal à 1, 2, 3 ou 4, ou éventuellement
o un groupe -(CH₂)_{q}-NR1- avec q représentant un nombre entier égal à 1, 2, 3 ou 4 et R1 tel que définis ci-dessus, R1 représentant avantageusement un groupe (C₁-C₆)alkyle, tel que méthyle.

Dans ce cas, X représentera avantageusement un groupement (C₁-C₆)alkyle, (C₂-C₆)alcényle, (C₂-C₆)alcynyle, [(C₁-C₆)alkyl]ₙ-hétéroaryl-[(C₁-C₆)alkyle]ₚ, -(CH₂)_{q}-NR1- ou -CO-(CH₂)_{q}-, avec n, p et q tels que définis ci-dessus, et de préférence avec n = p = 1. Le groupement hétéroaryle sera alors avantageusement un hétéroaryle comportant un seul cycle à 5 ou 6 chaînons et comprenant avantageusement un ou plusieurs, de préférence 1 à 3, atomes d'azote.

En particulier, X pourra représenter un groupement (C₁-C₆)alkyle, (C₂-C₆)alcényle, (C₂-C₆)alcynyle, [(C₁-C₆)alkyl]ₙ-hétéroaryl-[(C₁-C₆)alkyle]ₚ ou -CO-(CH₂)_{q}-, avec n, p et q tels que définis ci-dessus, et de préférence avec n = p = 1. Le groupement hétéroaryle sera alors avantageusement un hétéroaryle comportant un seul cycle à 5 ou 6 chaînons et comprenant avantageusement un ou plusieurs, de préférence 1 à 3, atomes d'azote.

En particulier, X pourra être choisi parmi les groupements suivants : -(CH₂)ₙ- avec n représentant 1, 2, 3, 4, 5 ou 6, -CH₂-CH(OH)-CH(OH)-CH₂-, -CH₂CH₂NCH₃-, -CO-CH₂-, -CH₂-CH=CH-CH₂-, -CH₂-C≡C-, 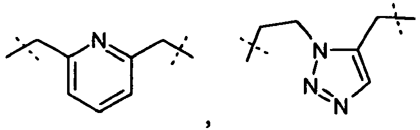 ou 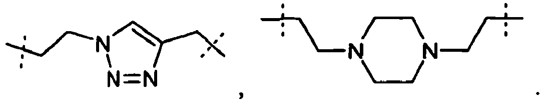 X pourra être choisi notamment parmi les groupements suivants : -(CH₂)ₙ- avec n représentant 1, 2, 3 ou 4, -CO-CH₂-, -CH₂-CH=CH-CH₂-, -CH₂-C≡C-,

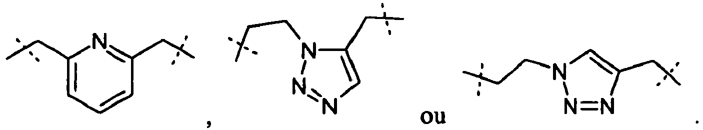

Selon un mode de réalisation particulier de l'invention, les composés de l'invention correspondent à des composés de formule (I) pour lesquels :
- a, b et 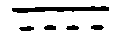 sont tels que définis précédemment,
- A représente un hétéroatome choisi parmi un atome d'azote ou d'oxygène, l'atome d'azote étant substitué par un atome d'hydrogène lorsque 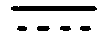 représente une liaison simple,
- B représente un groupe -CH₂-Y-, -C(=O)-NH- ou -CH(OH)-, avec Y représentant O, N-R1 ou un hétérocycle, R1 étant choisi parmi un atome d'hydrogène, un groupement (C₁-C₆)alkyle, (C₂-C₆)alcényle, -CH₂C(O)OR2 et -CH₂C(O)NHR2, avec R2 représentant un atome d'hydrogène, ou un groupe polyamino éventuellement substitué, et
- X représente :
   ■ lorsque représente une liaison double :
      un groupement =C(X1)-(O-X2)_{c}- avec X1 et X2 représentant, indépendamment l'un de l'autre, un groupement (C₁-C₆)alkyle ou (C₂-C₆)alcényle et c représentant 0 ou 1, ou
   ■ lorsque représente une liaison simple:
      - un groupement (C₁-C₆)alkyle éventuellement substitué par un ou plusieurs groupes OH ; (C₂-C₆)alcényle ; (C₂-C₆)alcynyle ; [(C₁-C₆)alkyl]n-hétérocycle-[(C₁-C₆)alkyle]ₚ ; [(C₁-C₆)alkyl]ₙ-hétéroaryl-[(C₁-C₆)alkyle]ₚ ; avec n et p représentant, indépendamment l'un de l'autre, 0 ou 1,
      - un groupement -CO-(CH₂)q- pour lequel q représente un nombre entier égal à 1, 2, 3 ou 4, ou
      - un groupement -(CH₂)q-NR4 pour lequel q représente un nombre entier égal à 1, 2, 3 ou 4 avec R4 représentant un atome d'hydrogène ou un groupe (C₁-C₆)akyle.

Selon un autre mode de réalisation particulier de l'invention, les composés de l'invention correspondent à des composés de formule (I) pour lesquels :
- a et b sont tels que définis précédemment,
- 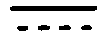 représente une liaison simple,
- A représente un atome d'oxygène,
- B représente un groupe -CH₂-Y-, avec Y représentant O, N-R1 ou un hétérocycle, R1 étant tel que défini précédemment et représentant avantageusement un atome d'hydrogène, un groupement (C₁-C₆)alkyle, (C₂-C₆)alcényle, -CH₂C(O)OR2 ou - CH₂C(O)NR2R2bis,
   avec R2 tel que défini précédemment et représentant avantageusement un atome d'hydrogène ou un groupement, polyamino éventuellement substitué, et
   R2bis tel que défini précédemment et représentant avantageusement un atome d'hydrogène, et
- X représente un groupement (C₁-C₆)alkyle, (C₂-C₆)alcényle, (C₂-C₆)alcynyle, [(C₁-C₆)alkyl]n-hétérocycle-[(C₁-C₆)alkyle]p, ou [(C₁-C₆)akyl]n-hétéroaryl-[(C₁-C₆)alkyle]p, avec n et p représentant, indépendamment l'un de l'autre, 0 ou 1.

Les composés de l'invention de formule (I) pourront être choisis plus particulièrement parmi les composés suivants :

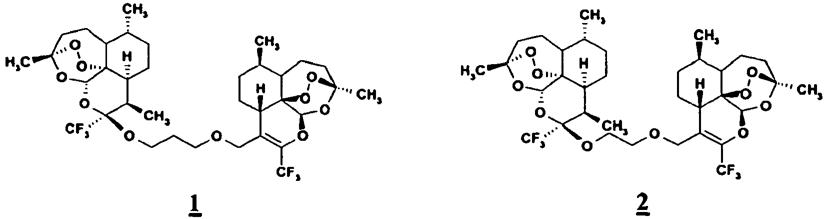

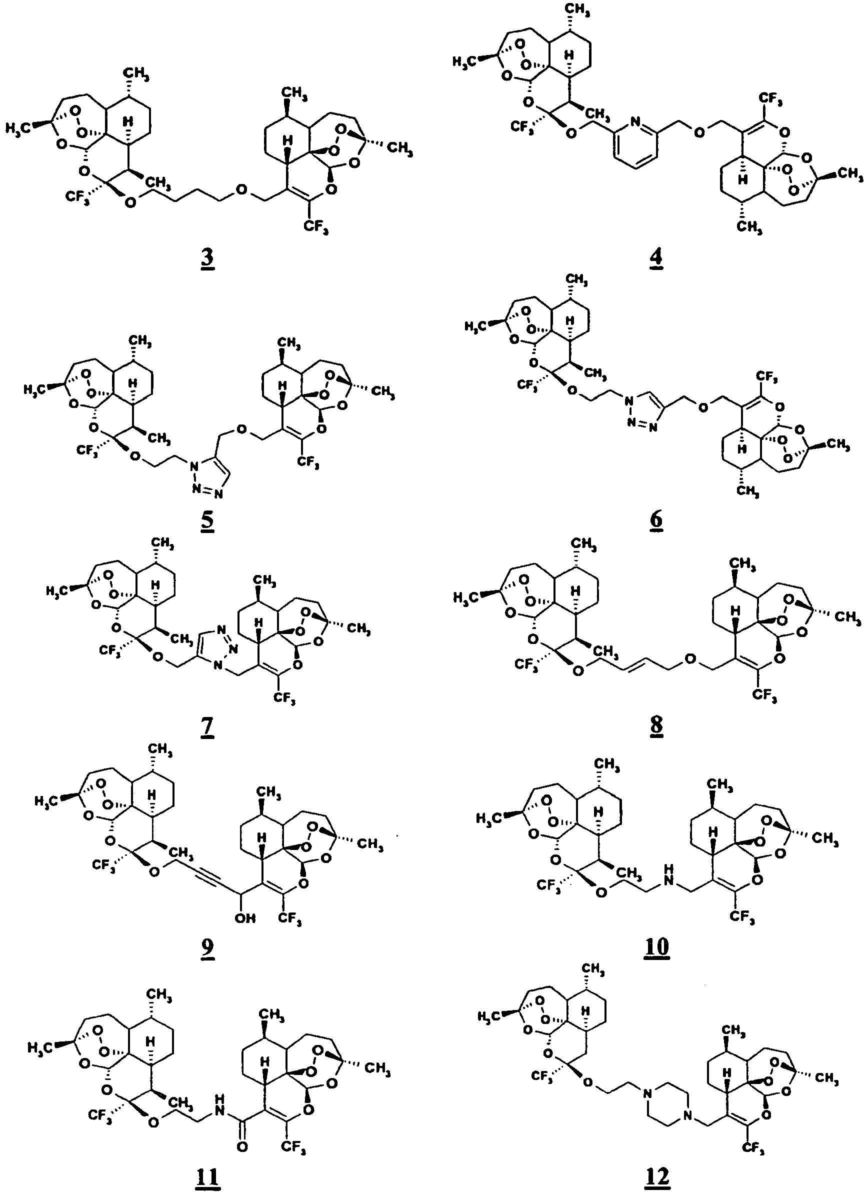

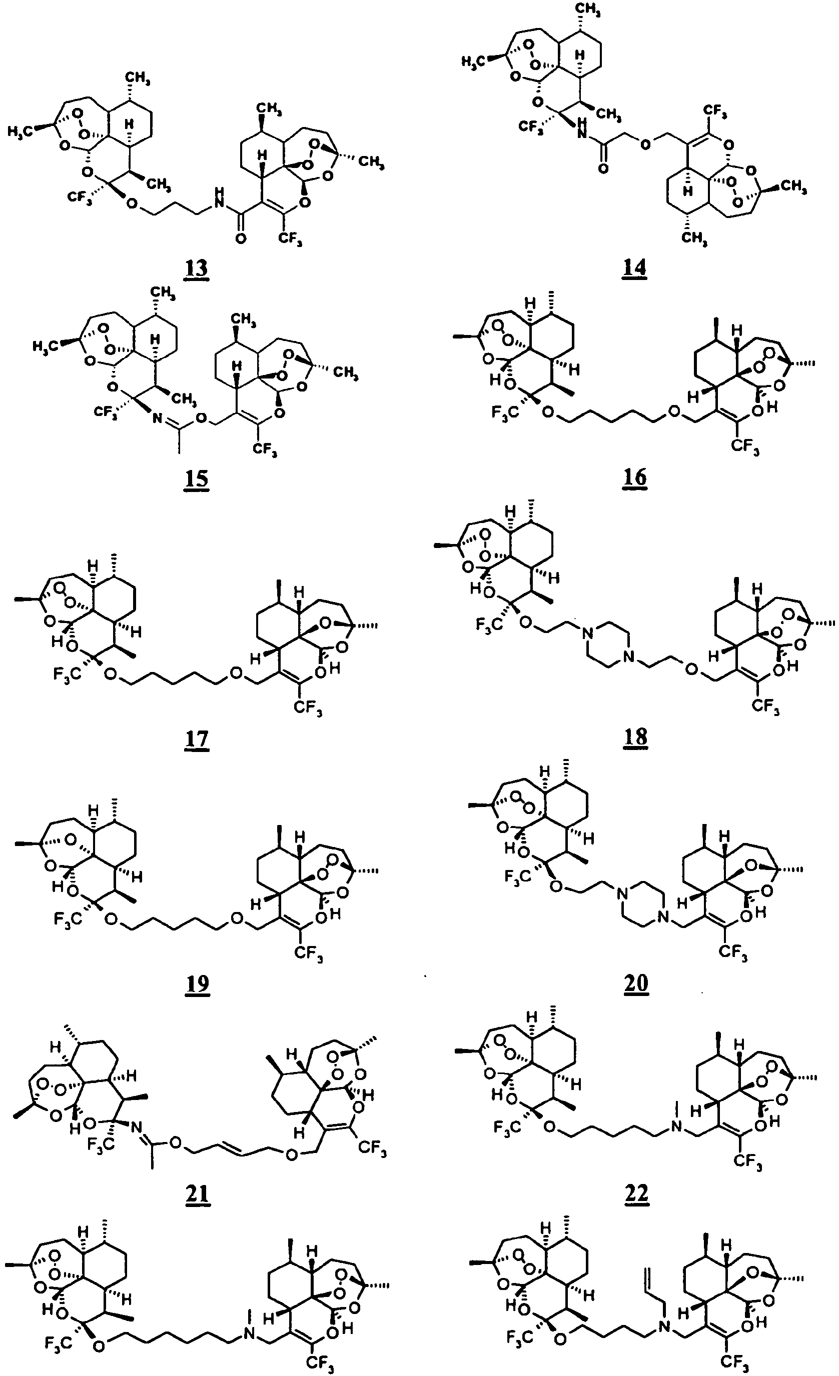

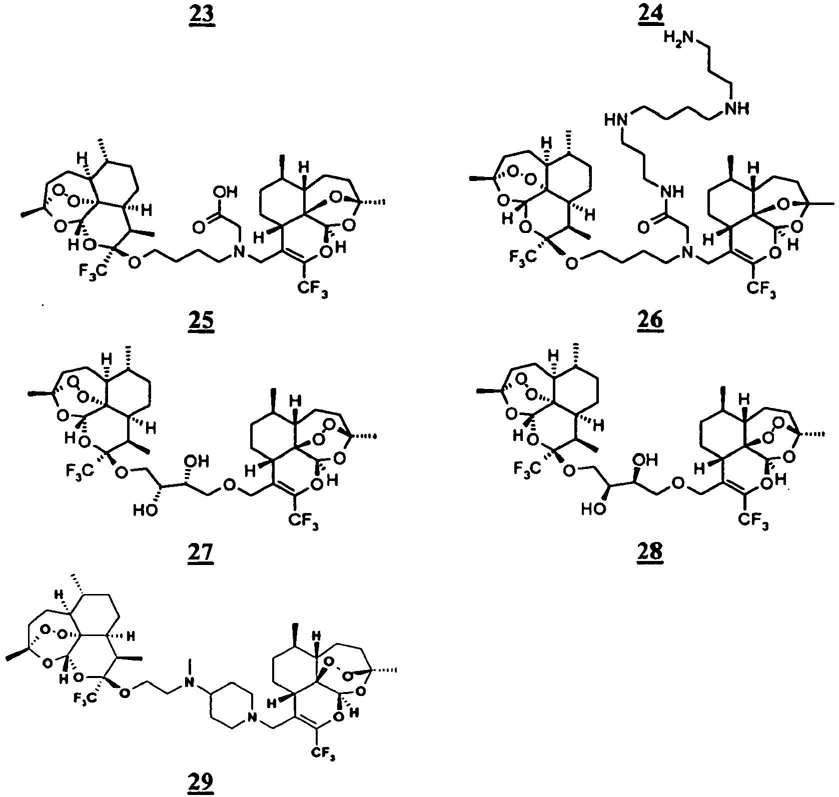

La présente invention a également pour objet un composé de formule (I) tel que défini ci-dessus pour son utilisation en tant que médicament, notamment destiné au traitement du cancer.

L'invention concerne également l'utilisation d'un composé de l'invention pour la fabrication d'un médicament destiné au traitement du cancer.

L'invention concerne aussi un composé de formule I pour l'utilisation dans une méthode pour le traitement du cancer, comprenant l'administration d'une quantité efficace d'au moins un composé de formule (I) tel que défini ci-dessus à un patient en ayant besoin.

La présente invention a également pour objet une composition pharmaceutique comprenant au moins un composé selon l'invention et au moins un véhicule pharmaceutiquement acceptable.

Les composés selon l'invention peuvent être administrés par voie orale, sublinguale, parentérale, sous-cutanée, intramusculaire, intraveineuse, transdermique, locale ou rectale, et de préférence par voie orale, intraveineuse ou sous-cutanée.

Dans les compositions pharmaceutiques de la présente invention pour l'administration orale, sublinguale, parentérale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration parentérale, sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier pourra se rendre compte lui-même.

Dans un mode de réalisation particulier, cette composition pourra comprendre en outre au moins un autre principe actif, avantageusement choisi parmi les agents anticancéreux.

A titre d'agent anticancéreux, on peut citer de manière non limitative la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

La présente invention a également pour objet une composition pharmaceutique comprenant :
(i) au moins un composé de formule (I) tel que défini ci-dessus, et
(ii) au moins un autre principe actif, notamment utile pour le traitement du cancer,
en tant que produits de combinaison pour une utilisation simultanée, séparée ou étalée dans le temps.

A titre de principe actif, on peut citer notamment, de façon non limitative, la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifene, l'octréotide ou le lanréotide.

La composition pharmaceutique telle que décrite ci-dessus peut être utile en particulier pour le traitement du cancer.

La présente invention concerne également l'utilisation d'une composition pharmaceutique comprenant :
(i) au moins un composé de formule (I) tel que défini ci-dessus, et
(ii) au moins un autre principe actif, notamment utile pour le traitement du cancer,
en tant que produits de combinaison pour une utilisation simultanée, séparée ou étalée dans le temps, pour la fabrication d'un médicament destiné au traitement du cancer.

Les composés de l'invention peuvent être préparés notamment à partir des intermédiaires **A, A₁, B, C, D, E, F, G, G₁** et **R** décrits ci-dessous. Ainsi, les composé de l'invention sont obtenus par couplage entre l'intermédiaire **G, G₁,** ou **R** et un intermédiaire **A, A₁, B, C, D,** **E** ou **F,** ce couplage pouvant être effectué en une ou plusieurs étapes.

### Intermédiaires A et B :

La synthèse de l'intermédiaire **A** bromo-trifluorométhylé de l'artémisinine et du dérivé hydroxylé **B** sont décrites dans J. Med. Chem., 47, 1423-33, (2004) et WO 03/035 651. Le protocole est indiqué sur le schéma réactionnel ci-dessous. 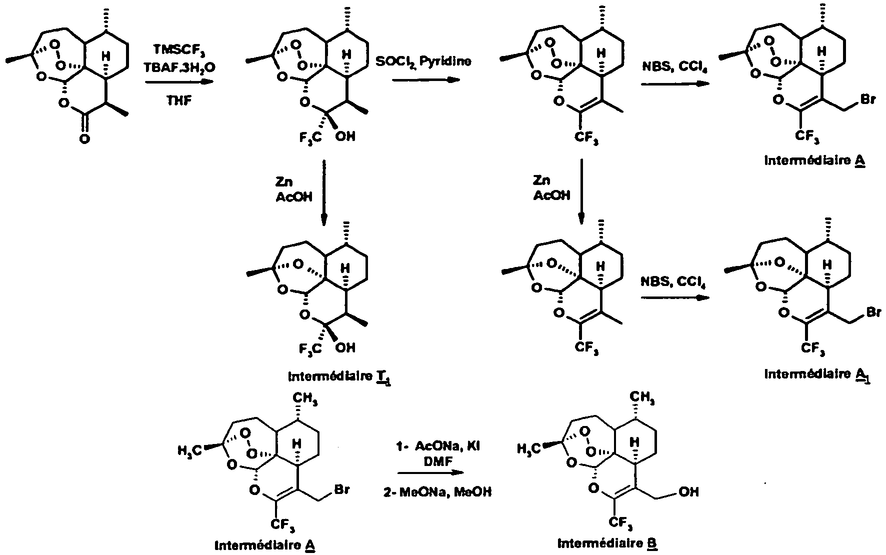 (TMS = triméthylsilyle ; TBAF = fluorure de tétrabutylammonium ; THF = tétrahydrofurane ; NBS = *N*-bromosuccinimide, Ac = acétyle ; Me = méthyle ; DMF = diméthylformamide)

### Intermédiaires C et D :

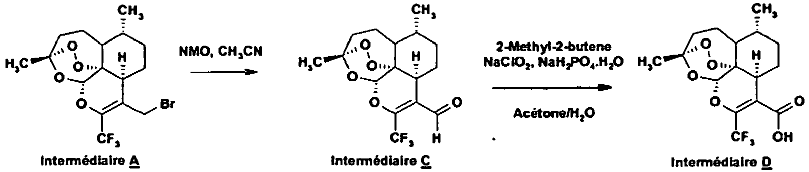

### Etape 1 : Synthèse de l'intermédiaire C

A une solution de **A** (0.83 g, 2.0 mmol) dans l'acétonitrile (20 mL) est ajouté l'oxyde de *N-*méthyl-morpholine (NMO) (1.08 g, 8.0 mmol, 4 éq.). Le mélange réactionnel est agité 1 heure à température ambiante puis condensé sous pression réduite. Le résidu est repris dans le dichlorométhane et lavé à l'eau. La phase organique est ensuite séchée sur sulfate de magnésium, filtrée et évaporée sous pression réduite. L'aldéhyde intermédiaire **C** ainsi obtenu (poudre blanche, 0.68 g, 98%) est directement engagé dans les réactions suivantes sans étape de purification supplémentaire.

### Etape 2 : Synthèse de l'intermédiaire D

A une solution de l'intermédiaire **C** (2.10 g, 6.0 mmol) dans un mélange acétone/eau (1.5/1, 75 mL) sont successivement additionnés le 2-méthyl-2-butène (3.2 mL, 30.1 mmol, 5 éq.), le phosphate de sodium monohydrate (2.50 g, 18.1 mmol, 3 éq.) puis le chlorite de sodium (1.64 g, 18.1 mmol, 3 éq.). Le mélange réactionnel est agité 18 heures à température ambiante puis condensé sous pression réduite. Après dilution à l'acétate d'éthyle, la phase organique est lavée à l'eau puis à l'aide d'une solution saturée de chlorure de sodium et séchée sur sulfate de magnésium. Après filtration, les solvants sont évaporés sous pression réduite. Le résidu est purifié par chromatographie sur gel de silice (cyclohexane/acétate d'éthyle 80:20) ; l'intermédiaire **D** est isolé avec un rendement de 54% (poudre blanche, 1.2 g).

### Intermédiaires E et F :

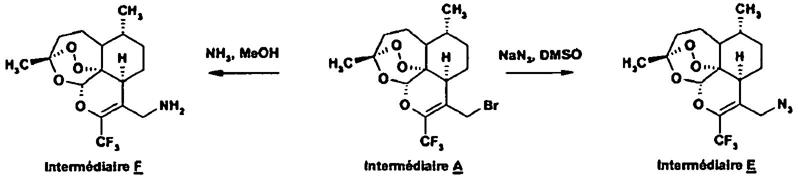

### Synthèse de l'intermédiaire E :

A une solution de l'intermédiaire **A** (0.63 g, 2.5 mmol) dans le diméthylsulfoxyde (DMSO) (10 mL) est additionné l'azoture de sodium (0.14 g, 2.25 mmol, 1.5 éq.). Le mélange réactionnel est agité à température ambiante pendant 1.5 heures. Il est ensuite versé dans l'eau et l'extraction est réalisée à l'acétate d'éthyle. La phase organique est séchée sur sulfate de magnésium, filtrée et les solvants sont évaporés sous pression réduite. L'azoture intermédiaire **E** ainsi obtenu (poudre blanche, 0.56 g, 100%) est directement engagé dans les réactions suivantes sans étape de purification supplémentaire.

### Synthèse de l'intermédiaire F :

L'intermédiaire **A** (1.03 g, 2.5 mmol) est dissous dans une solution d'ammoniac dans le méthanol (7N, 10 mL). Le mélange réactionnel est agité à température ambiante pendant 3 heures puis évaporé sous pression réduite. Le résidu est purifié par chromatographie sur gel de silice (dichlorométhane/méthanol 95:5 puis 90:10) ; l'intermédiaire **F** est isolé avec un rendement de 59% (poudre jaune pâle, 0.52 g).

### Intermédiaires G et R :

La synthèse de l'intermédiaire **G** nécessaire à la préparation des composés de l'invention est décrite dans Org. Lett., 4, 757-759, (2002), dont le protocole est indiqué sur le schéma réactionnel ci-dessous.

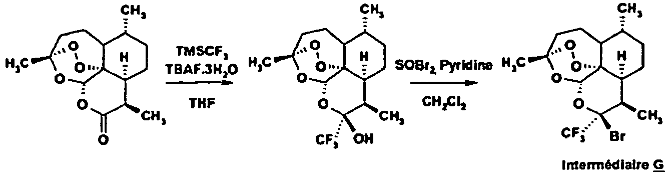

La synthèse de l'intermédiaire **R** à partir de l'intermédiaire **G** est décrite dans le procédé de synthèse du composé 14 ci-après.

### Intermédiaires A₁ et G₁ :

Les intermédiaires **A₁** et **G₁** possèdent un pont endoperoxyde réduit dans le noyau artémisinine.

### Synthèse de l'intermédiaire A₁ :

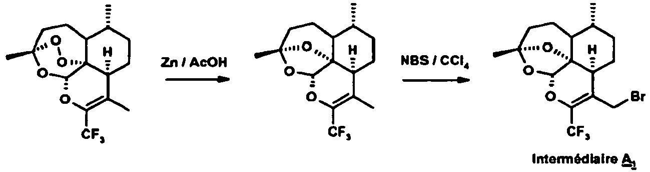

A une solution du dérivé trifluorométhylé décrit dans J. Med. Chem., 47, 1423-33, (2004) (300 mg, 0.897 mmol) dans l'acide acétique 100 % (8 mL) est ajouté du zinc en poudre (264 mg, 0.403 mmol, 4.5 éq.). Après 4h30 d'agitation à température ambiante, le milieu réactionnel est filtré sur silice puis concentré sous pression réduite. Le brut réactionnel est purifié par chromatographie sur gel de silice avec un gradient d'éluant (éther de pétrole/éther diéthylique : 100 à 90/10). Le dérivé au pont réduit est obtenu avec un rendement de 90 % (259 mg).

A une solution de dérivé au pont réduit (259 mg, 0.813 mmol) dans le tétrachlorure de carbone (10 mL) est ajouté le N-bromosuccinimide (NBS) (290 mg, 1.62 mmol, 2 éq.). Le milieu réactionnel est porté au reflux instantanément. Après 1h à cette température, le mélange réactionnel est dilué avec du dichlorométhane (5 mL), lavé avec une solution saturée de NaHCO₃ (2 x 5 mL) puis avec une solution de NaCl saturée (5 mL). La phase organique est séchée sur MgSO₄, filtrée et concentrée sous pression réduite. Le brut réactionnel est purifié par chromatographie sur gel de silice avec un gradient d'éluant (éther de pétrole/éther diéthylique : 100 à 90/10). L'intermédiaire **A₁** est obtenu avec 60% de rendement (186 mg).

### Synthèse de l'intermédiaire G₁ :

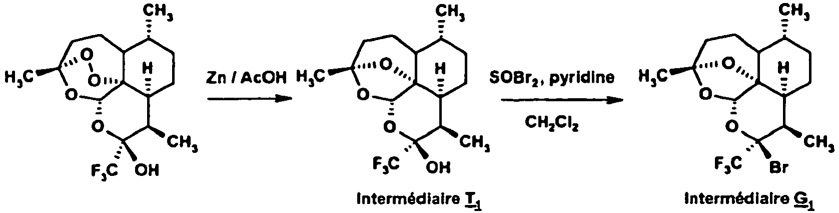

A une solution d'alcool trifluorométhylé décrit dans J. Med. Chem., 47, 1423-33, (2004) (1.5 g, 4.25 mol) dans l'acide acétique 100 % (37.5 mL) est ajouté du zinc en poudre (1.25 g, 19.15 mmol, 4.5 éq.). Après 4h30 d'agitation à température ambiante, le milieu réactionnel est filtré sur silice puis est concentré sous pression réduite. L'intermédiaire **T₁** au pont réduit est obtenu avec un rendement quantitatif (1.42 g).

Ce dernier (1.42 g, 4.23 mmol) est mis en solution dans le dichlorométhane (26 mL), dans lequel sont ajoutés, à 0°C et successivement : la pyridine (690 µL, 8.46 mmol, 2 éq.) puis le bromure de thionyle (972 µL, 8.46 mmol, 2 éq.). Le mélange réactionnel est laissé revenir à température ambiante et après 4h30 d'agitation est hydrolysé avec une solution de NaCl saturée. La phase organique est basifiée avec une solution saturée de NaHCO₃ (2 x 10 mL), lavée avec une solution de NaCl saturée (10 mL), séchée sur MgSO₄, filtrée et concentrée sous pression réduite. L'intermédiaire **G₁** est obtenu avec 80 % de rendement (1.34 g).

La présente invention a ainsi pour objet un procédé de préparation d'un composé de formule (I) tel que défini ci-dessus, caractérisé en ce que le composé de formule (I) est obtenu par couplage d'un composé de formule (II) suivante : 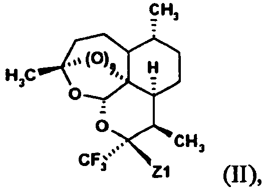 pour laquelle a est tel que défini précédemment et Z1 représente un atome d'halogène, de préférence un atome de brome, ou un groupement -OH, -NH₂ ou -NHR1, R1 étant tel que défini ci-dessus, de préférence Z1 représentant un atome d'halogène ou un groupement - OH ou -NH₂, avec un composé de formule (III) suivante : 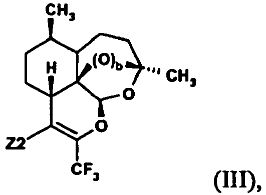 pour laquelle b est tel que défini précédemment et Z2 représente un groupement -CH₂OH, -CHO, -COOH, -CH₂N₃, -CH₂NH₂ ou -CH₂Hal, où Hal représente un atome d'halogène, de préférence un atome de brome.

Le composé de formule (I) ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Le couplage pourra comprendre une ou plusieurs étapes réactionnelles réalisées par des techniques bien connues de l'homme du métier.

Selon un premier mode de réalisation particulier, un composé de formule (I) pour lequel B = -CH₂Y- ou -C(=O)Y-, avec Y tel que défini ci-dessus, peut être préparé par couplage entre les composés (II) et (III) et un composé de formule (IV) suivante :

Z3-X-Z4 (IV),

avec :
X tel que défini ci-dessus,
Z3 représentant un atome d'halogène, tel qu'un atome de brome ou de chlore, ou un groupement -OH, -NHR1, -SH, -N₃, -C(=O)R4 ou un hétérocycle comportant au moins un groupement NH intracyclique, R1 étant tel que défini ci-dessus et R4 représentant un atome d'hydrogène ou un groupement (C₁-C₆)alkyle, et
Z4 représentant un atome d'halogène, tel qu'un atome de brome ou de chlore, ou un groupement -OH, -NHR1, -SH, -N₃ ou un hétérocycle comportant au moins un groupement NH intracyclique, R1 étant tel que défini ci-dessus.

Dans ce cas, la fonction Z2 portée par le composé de formule (III) sera de préférence un groupe -CH₂OH, -CH₂NH₂ ou -CH₂Hal (pour donner accès aux composé (I) avec B = -CH₂O-, -CH₂NR1- ou -CH₂S- ou -COOH (pour donner accès aux composé (I) avec B = -C(=O)O-, -C(=O)NR1- ou -C(=O)S-, et encore de préférence sera un groupe - CH₂Hal ou -COOH.

Dans le cas où Z3 et/ou Z4 représente un hétérocycle, la fonction active qui réagira dans l'étape de couplage sera bien évidemment le groupement NH intracyclique. L'hétérocycle sera de préférence à 5 ou 6 chaînons, tel qu'un groupe morpholinyle, pipéridinyle ou pypérazinyle.

Le composé de formule (I) est alors obtenu en deux étapes, d'une part (1) par couplage de la fonction Z1 du composé (II) avec la fonction Z3 du composé (IV), et d'autre part (2) par couplage de la fonction Z2 du composé (III) avec la fonction Z4 du composé (IV). L'ordre de réalisation de ces deux étapes (1) et (2) est indifférent, ces deux étapes de couplage étant réalisées par des techniques bien connues de l'homme du métier.

Si nécessaire, la fonction Z3 ou Z4, qui ne doit pas réagir dans la première étape du couplage, pourra être préalablement protégée puis déprotégée une fois cette étape réalisée. Notamment, si une fonction amine libre NH₂ est désirée, elle pourra être obtenue à partir du dérivé halogéné ou d'un azoture N₃. Une fonction amine NH ou NH₂ pourra être également protégée sous forme de carbamate, notamment par un groupement Boc. De même, un atome d'halogène pourra être obtenu par halogénation d'un groupe OH (par exemple en présence de N-bromosuccinimide pour obtenir un atome de brome).

De même, il peut être nécessaire d'activer certaines fonctions intervenant dans le couplage (Z1, Z2, Z3 ou Z4). Notamment, dans le cas d'une fonction acide COOH, celle-ci peut être activée sous forme d'un chlorure d'acyle COCI.

Des étapes supplémentaires de fonctionnalisation peuvent également être effectuées (voir exemples 25 et 26).

Le composé de formule (IV) pourra être soit commercial, soit préparé par des procédés bien connus de l'homme du métier.

Selon un premier aspect, Z3 représente un atome d'halogène, un hétérocycle ou un groupement -OH, -NHR1, -SH ou -N₃, ce qui permet d'accéder à des composés de formule (I) pour lesquels 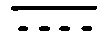 représente une liaison simple.

Selon un second aspect, Z3 représente un groupement -C(=O)R4 et Z1 représente un groupement -NH₂, ce qui permet d'accéder à des composés de formule (I) pour lesquels 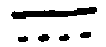 représente une liaison double et donc A représente un atome d'azote.

Ce premier mode de réalisation particulier de préparation des composés de l'invention est illustré notamment avec la synthèse des composés 1, 2, 3, 4, 10, 11, 12, 13, 14, 16, 17, 18, 19, 20, 22, 23, 24, 25, 26 et 29.

Selon un deuxième mode de réalisation particulier, un composé de formule (I) pour lequel B = -CH(OR3)-, avec R3 tel que défini ci-dessus, peut être préparé par couplage entre les composés (II) et (III), avec Z2 = -CHO, et un composé de formule (V) suivante :

Z5-X-Z6 (V),

avec :
X tel que défini ci-dessus,
Z5 représentant un atome d'halogène, tel qu'un atome de brome ou de chlore, ou un groupement -OH, -NHR1, -SH, -N₃, -C(=O)R4 ou un hétérocycle comportant au moins un groupement NH intracyclique, R1 et R4 étant tel que défini ci-dessus, et
Z6 représentant un atome d'hydrogène acide ou un atome d'halogène.

Par « atome d'hydrogène acide », on entend, au sens de la présente invention, un atome d'hydrogène qui peut être déplacé par une base. Un tel atome d'hydrogène acide peut être en particulier un atome d'hydrogène acétylénique.

Dans le cas où Z5 représente un hétérocycle, la fonction active qui réagira dans l'étape de couplage sera bien évidemment le groupement NH intracyclique. L'hétérocycle sera de préférence à 5 ou 6 chaînons, tel qu'un groupe morpholinyle, pipéridinyle ou pypérazinyle.

Ce procédé comprendra en particulier les étapes successives suivantes :
(i) mise en réaction du composé (V) avec le composé (II) pour donner le composé (VI) suivant : 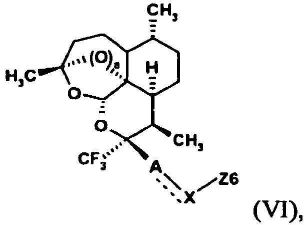 avec a, A, X et Z6 tels que définis précédemment,
(ii) formation, à partir du composé (VI) obtenu à l'étape (i) précédente, du composé organométallique (VII) suivant : 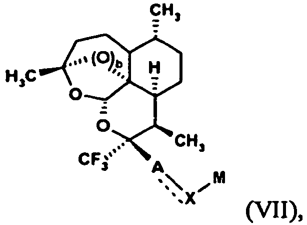 avec b, A et X tels que définis précédemment et M représentant un métal alcalin tel qu'un lithium ou un halogénure de métal alcalino terreux tel qu'un chlorure ou un bromure de magnésium,
(iii) mise en réaction du composé (VII) obtenu à l'étape (ii) précédente avec un composé de formule (III) pour lequel Z2 = -CHO pour donner un composé de formule (I) pour lequel B = -CH(OH)-,
(iv) éventuellement substitution de la fonction hydroxyle du composé de formule (I) obtenu à l'étape (iii) précédente pour donner un composé de formule (I) pour lequel B = - CH(OR3)- avec R3 ≠ H, et
(v) séparation du milieu réactionnel du composé de formule (I) obtenu à l'étape (iv) ou (v).

Le composé organométallique (VII) peut être formé à partir du composé (VI) pour lequel Z6 = H par réaction avec une base organométallique telle qu'un alkylithium (comme n-Bu-Li) ou un bromure ou un chlorure d'alkylmagnésium.

Lorsque le composé (VI) comporte un groupement Z6 représentant un atome d'halogène tel qu'un atome de brome, un échange halogène-métal peut donner accès au composé (VII) souhaité par mise en réaction du composé (VI) avec un métal tel que le lithium ou le magnésium.

Le composé de formule (V) pourra être soit commercial, soit préparé par des procédés bien connus de l'homme du métier.

Selon un premier aspect, Z5 représente un atome d'halogène, un hétérocycle ou un groupement -OH, -NHR1, -SH ou -N₃, ce qui permet d'accéder à des composés de formule (I) pour lesquels 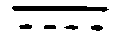 représente une liaison simple. Dans ce cas, Z5 représente de préférence un groupement -OH, -NHR1 ou -SH et Z1 représente un atome d'halogène tel qu'un atome de brome.

Selon un second aspect, Z5 représente un groupement -C(=O)R4 et Z1 représente un groupement -NH₂, ce qui permet d'accéder à des composés de formule (I) pour lesquels 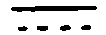 représente une liaison double et donc A représente un atome d'azote.

Ce deuxième mode de réalisation particulier de préparation des composés de l'invention est illustré notamment avec la synthèse du composé 9.

Selon un troisième mode de réalisation particulier, le couplage est réalisé par métathèse croisées des alcènes ou des alcynes comme cela est illustré avec la préparation des composés 8 et 21. Dans ce cas, une ou plusieurs étapes préalables devront être effectuées pour introduire un groupement portant une double ou une triple liaison terminale à la place des groupements Z1 et Z2 des composés (II) et (III).

De plus, des étapes supplémentaires de fonctionnalisation peuvent être effectuées, notamment des étapes de fonctionnalisation de la double ou triple liaison formée au cours de la métathèse (voir exemples 27 et 28).

Ainsi, un composé de formule (I) pour lequel :
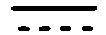 représente une liaison simple, A représente un hétéroatome, B = -CH₂O-, - CH₂NR1- ou -CH₂S- et X = -(CH₂)_{y}-Z7-(CH₂)_{z}-, avec Z7 représentant le groupe -CH=CHou -C=C- et y et z représentant, indépendamment l'un de l'autre, un nombre entier compris entre 0 et 4, avec y + z ≤ 4, ou
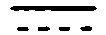 représente une liaison double, A représente N, B = -CH₂O-, -CH₂NR1- ou -CH₂Set X représente =C((C₁-C₆)alkyle)-O-(CH₂)_{y}-Z7-(CH₂)₂-, avec Z7, y et z tels que définis ci-dessus,
peut être obtenu par une réaction de métathèse croisée entre le composé de formule (VIII) suivante : 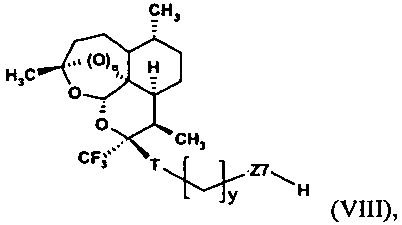 avec a, Z7 et y tels que définis précédemment, et T représentant A ou -N=C((C₁-C₆)alkyle)-O-,
   et le composé de formule (IX) suivante : 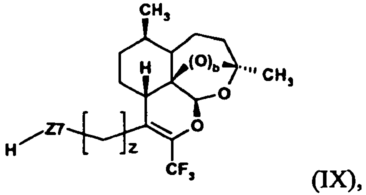
avec b, B, Z7 et z tels que définis précédemment.

Cette réaction sera réalisée en présence d'un catalyseur de métathèse tel que le catalyseur de Grubb de 1^{ère} ou 2^{nde} génération.

De préférence, Z7 représente le groupement -CH=CH-.

Le composé (VIII) pourra être préparé par réaction d'un composé de formule (II), pour lequel Z1 représente de préférence un atome d'halogène, avec un composé de formule Z7-(CH₂)_{y}-R, où Z7 et y sont tels que définis ci-dessus et R représente un groupement OH, NHR1 ou SH, R1 étant tel que défini ci-dessus.

Le composé (IX) pourra être préparé par réaction d'un composé de formule (III), pour lequel Z2 représente de préférence un atome d'halogène, avec un composé de formule Z7-(CH₂)_{z}-R', où Z7 et z sont tels que définis ci-dessus et R' représente un groupement OH, NHR1 ou SH, R1 étant tel que défini ci-dessus.

Selon un quatrième mode de réalisation particulier, le couplage est réalisé par une réaction de cycloaddition [4+2] ou [3+2], comme cela est illustré avec la préparation des composés 5, 6 et 7. Dans ce cas, une ou plusieurs étapes préalables devront être effectuées pour introduire des groupements adaptés à la place des groupements Z1 et Z2 des composés (II) et (III), comme un alcène ou un alcyne pour un des deux groupements et un diène ou un dipôle 1,3 tel qu'un groupe N₃ pour l'autre groupement.

Selon un cinquième mode de réalisation particulier, un composé de formule (I) pour lequel -A-X-B- représente -N=C((C₁-C₅)alkyle)-O-CH₂- peut être préparé par couplage en milieu basique d'un composé de formule (III) pour lequel Z2 représente un atome d'halogène tel qu'une atome de brome avec un composé de formule (X) suivante : 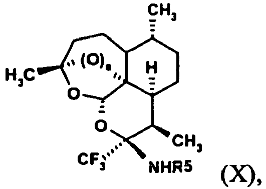 avec a tel que défini ci-dessus et R5 représentant un groupement acyle de formule -C(=O)-(C₁-C₅)alkyle.

De préférence, cette réaction est réalisée en présence de NaH comme base, en présence notamment de KI.

Le composé de formule (X) peut être préparé par acylation à partir d'un composé de formule(II) pour lequel Z1 = NH₂ ou selon le protocole décrit pour la synthèse de l'intermédiaire **V.**

Ce deuxième mode de réalisation particulier de préparation des composés de l'invention est illustré notamment avec la synthèse du composé 15.

Dans tous les procédés décrits ci-dessus, des étapes supplémentaires de substitution et/ou activation et/ou protection/déprotection, bien connues de l'homme du métier, pourront être réalisées si nécessaire.

La présente invention sera mieux comprise à la lumière des exemples non limitatifs qui suivent.

### EXEMPLES

### EXEMPLE 1 : Synthèse des composés de l'invention

### Synthèse des composés 1, 2, 3, 4, 16, 17, 18 et 19

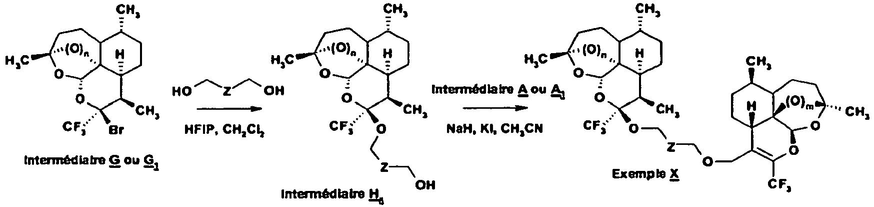

(Z représente une liaison simple ou un groupement -CH₂-, -(CH₂)₂-, -(CH₂)₃-, pyridinyle ou 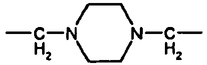 dans les exemples 1 à 4 et 16 à 19)

### Méthode générale A :

1^{ère} étape: A une solution de l'intermédiaire **G** ou **G₁** (1 éq.) dans le dichlorométhane (c=0.14) sont ajoutés successivement le diol (HO-CH₂-Z-CH₂OH) (10 éq.) et l'hexafluoroisopropanol (HFIP) (5 éq.). Le mélange réactionnel est ensuite agité à température ambiante pendant 5 heures avant dilution au dichlorométhane. La phase organique est lavée avec une solution d'hydrogénocarbonate de sodium, séchée sur sulfate de magnésium. Après filtration, les solvants sont évaporés sous pression réduite. Le brut obtenu est purifié par chromatographie sur gel de silice (cyclohexane/acétate d'éthyle) pour conduire à l'intermédiaire **Hₓ.**

Intermédiaire **H₁ :** obtenu avec un rendement de 71% sous forme d'une poudre blanche à partir du 1,3-propanediol et l'intermédiaire **G** (cyclohexane/acétate d'éthyle 85:15 à 70:30).

Intermédiaire **H₂ :** obtenu avec un rendement de 71 % sous forme d'une poudre blanche à partir du 1,2-éthanediol et l'intermédiaire **G** (cyclohexane/acétate d'éthyle 90:10). Intermédiaire **H₃ :** obtenu avec un rendement de 25% sous forme d'une huile translucide à partir du 1,4-butanediol et l'intermédiaire **G** (cyclohexane/acétate d'éthyle 80:20). Intermédiaire **H₄** : obtenu avec un rendement de 39% sous forme d'une huile translucide à partir du 2,6-pyridinediméthanol et l'intermédiaire **G** (cyclohexane/acétate d'éthyle 95:5 à 75:25).

Intermédiaire **H₅ :** obtenu avec un rendement de 82% sous forme d'une huile incolore à partir du 1,5-pentanediol et l'intermédiaire **G** (cyclohexane/acétate d'éthyle 100 à 80:20). Intermédiaire **H₆ :** obtenu avec un rendement de 61% sous forme d'une huile jaune à partir du 1,6-hexanediol et l'intermédiaire **G** (cyclohexane/acétate d'éthyle 95:5 à 80:20). Intermédiaire **H₇ :** obtenu avec un rendement de 49% sous forme d'une mousse jaune à partir de la 1,4-bis-(2-hydroxyéthyl)pipérazine et l'intermédiaire **G** (dichlorométhane/méthanol 100:0 à 90:10).

Intermédiaire **H₈ :** obtenu avec un rendement de 22% sous forme d'une huile à partir de 1,5-pentanediol et l'intermédiaire **G₁** (cyclohexane/acétate d'éthyle 100:0 à 60:40). 2^{nde} étape : A une suspension dans l'acétonitrile (ou dans le diméthylsulfoxyde) (c=0.1) de NaH (60% dans l'huile, 2.5 éq.) est ajouté l'intermédiaire **Hₓ** (1 éq.) ; après 10 minutes d'agitation, est ajoutée à ce mélange, une solution dans l'acétonitrile (ou dans le diméthylsulfoxyde) (c=0.1) de l'intermédiaire **A** ou **A₁** (1.25 éq.) et d'iodure de potassium (en quantité catalytique). Le milieu réactionnel est agité à température ambiante pendant 1 à 5 heures avant dilution à l'acétate d'éthyle. La phase organique est lavée avec une solution d'hydrogénocarbonate de sodium, séchée sur sulfate de magnésium. Après filtration, les solvants sont évaporés sous pression réduite. Le brut obtenu est purifié par chromatographie sur gel de silice (cyclohexane/acétate d'éthyle) pour conduire au dimère attendu.

Exemple **1** : obtenu avec un rendement de 45% sous forme d'une poudre blanche à partir de l'intermédiaire **H₁** et de l'intermédiaire **A** (cyclohexane/acétate d'éthyle 95:5 à 70:30). Exemple **2** : obtenu avec un rendement de 40% sous forme d'une poudre blanche à partir de l'intermédiaire **H₂** et de l'intermédiaire **A** (cyclohexane/acétate d'éthyle 90:10). Exemple **3** : obtenu avec un rendement de 20% sous forme d'une poudre blanche à partir de l'intermédiaire **H₃** et de l'intermédiaire **A** (cyclohexane/acétate d'éthyle 95:5). **Exemple** 4 : obtenu avec un rendement de 30% sous forme d'une poudre blanche à partir de l'intermédiaire **H₄** et de l'intermédiaire **A** (cyclohexane/acétate d'éthyle 95:5 à 90:10). Exemple **16** : obtenu avec un rendement de 45% sous forme d'une poudre blanche à partir de l'intermédiaire **H₅** et de l'intermédiaire **A** (cyclohexane/acétate d'éthyle 85:15). Exemple **17** : obtenu avec un rendement de 40% sous forme d'une poudre blanche à partir de l'intermédiaire **H₆** et de l'intermédiaire **A₁** (cyclohexane/acétate d'éthyle 75:2). **Exemple 18 :** obtenu avec un rendement de 58% sous forme d'une poudre blanche à partir de l'intermédiaire **H₇** et de l'intermédiaire **A** (dichlorométhane/méthanol 97:3).

Exemple **19 :** obtenu avec un rendement de 1% sous forme d'une huile incolore à partir de l'intermédiaire **H₈** et de l'intermédiaire **A** (cyclohexane/acétate d'éthyle 98:2 à 70:30).

### Synthèse des composés 5 et 6

### Synthèse de l'intermédiaire J :

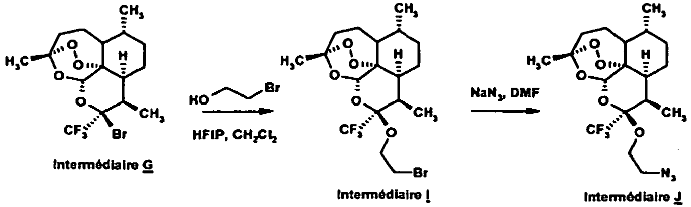

Etape 1 : A une solution de l'intermédiaire **G** (0.83 g, 2.0 mol) dans le dichlorométhane (14 mL) sont ajoutés successivement le 2-bromo-éthanol (1.41 mL, 20.0 mmol, 10 éq.) et l'hexafluoroisopropanol (1.05 mL, 10.0 mmol, 5 éq.). Le mélange réactionnel est ensuite agité à température ambiante pendant 2 heures avant dilution au dichlorométhane. La phase organique est lavée avec une solution d'hydrogénocarbonate de sodium puis séchée sur sulfate de magnésium. Après filtration, les solvants sont évaporés sous pression réduite. Le brut obtenu est purifié par chromatographie sur gel de silice (cyclohexane/acétate d'éthyle 95:5) ; l'intermédiaire **I** est isolé avec un rendement de 40% (poudre jaune pâle, 0.365 g).

Etape 2: A une solution de l'intermédiaire **I** (0.07 g, 0.15 mmol) dans le diméthylsulfoxyde (2 mL) est ajouté de l'azoture de sodium (0.02 g, 0.3 mmol, 2 éq.). Le mélange réactionnel est agité à température ambiante pendant 1 heure. Après dilution à l'acétate d'éthyle, la phase organique est lavée à l'eau puis séchée sur sulfate de magnésium. Après filtration, les solvants sont évaporés sous pression réduite et l'intermédiaire **J** obtenu quantitativement, est engagé directement dans l'étape suivante (poudre blanche).

### Synthèse de l'intermédiaire K :

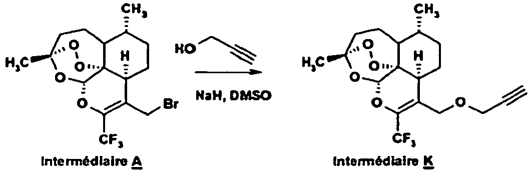

A une suspension dans le diméthylsulfoxyde (DMSO) (4 mL) de NaH (60% dans l'huile, 0.04 g, 1.0 mmol, 1 éq.) est ajouté l'alcool propargylique (64 µL, 1.0 mmol, 1 éq.) ; après 5 minutes d'agitation, est ajoutée à ce mélange, une solution dans le diméthylsulfoxyde (2 mL) de l'intermédiaire **A** (0.41 g, 1.0 mmol). Le milieu réactionnel est agité à température ambiante pendant 1.5 heures avant dilution à l'acétate d'éthyle. La phase organique est lavée avec une solution d'hydrogénocarbonate de sodium puis séchée sur sulfate de magnésium. Après filtration, les solvants sont évaporés sous pression réduite. Le brut obtenu est purifié par chromatographie sur gel de silice (cyclohexane/acétate d'éthyle 95:5) ; l'intermédiaire **K** est isolé avec un rendement de 80% (huile incolore, 0.31 g).

### Couplage des intermédiaires J et K :

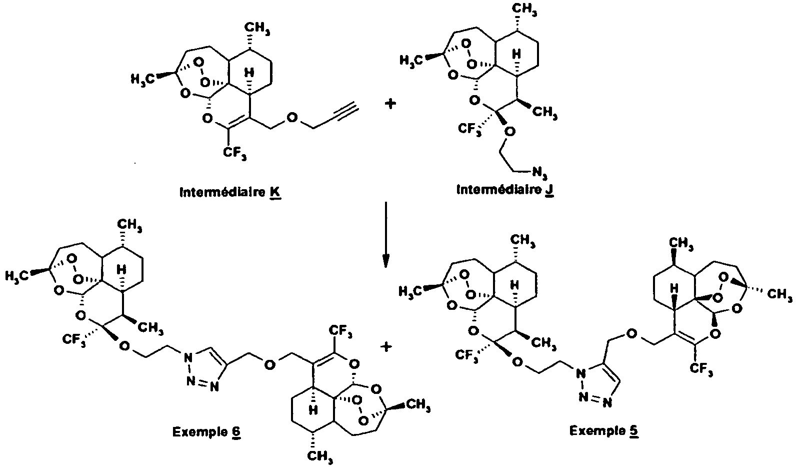

Un mélange de l'intermédiaire **J** (0.063 g, 0.15 mmol, 1 éq.) et de l'intermédiaire **K** (0.062 g, 0.15 mmol, 1 éq.) est chauffé à 90°C pendant 4 heures dans un tube scellé. Après retour à température ambiante, le milieu réactionnel est purifié par chromatographie sur gel de silice (cyclohexane/acétate d'éthyle 90:10 à 75:25) ; le composé **5**, moins polaire, et le composé **6** sont isolés avec un rendement global de 68% (poudre blanche, respectivement 0.035 g et 0.048 g).

### Synthèse du composé 7

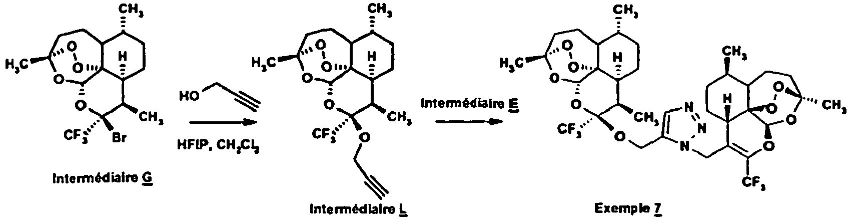

Etape 1 : A une solution de l'intermédiaire **G** (0.207 g, 0.5 mol) dans le dichlorométhane (3.5 mL) sont ajoutés successivement l'alcool propargylique (295 µL, 5.0 mmol, 10 éq.) et l'hexafluoroisopropanol (HFIP) (263 µL, 2.5 mmol, 5 éq.). Le mélange réactionnel est ensuite agité à température ambiante pendant 2 heures avant dilution au dichlorométhane. La phase organique est lavée avec une solution d'hydrogénocarbonate de sodium, séchée sur sulfate de magnésium. Après filtration, les solvants sont évaporés sous pression réduite. Le brut obtenu est purifié par chromatographie sur gel de silice (cyclohexane/acétate d'éthyle 97:3) ; l'intermédiaire **L** est isolé avec un rendement de 68% (poudre blanche, 0.133 g).

Etape 2 : Un mélange de l'intermédiaire **E** (0.056 g, 0.15 mmol, 1 éq.) et de l'intermédiaire **L** (0.058 g, 0.15 mmol, 1 éq.) est chauffé à 90°C pendant 16 heures dans un tube scellé. Après retour à température ambiante, le milieu réactionnel est purifié par chromatographie sur gel de silice (cyclohexane/acétate d'éthyle 80:20) ; le composé **7** est isolé avec un rendement global de 28% (poudre blanche, 0.032 g).

### Synthèse du composé 8

### Synthèse de l'intermédiaire M :

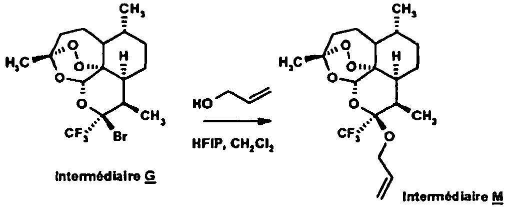

A une solution de l'intermédiaire **G** (0.5 g, 1.2 mmol) dans le dichlorométhane (7 mL) sont ajoutés successivement l'alcool allylique (823 µL, 12.0 mmol, 10 éq.) et l'hexafluoroisopropanol (634 µL, 6.0 mmol, 5 éq.). Le mélange réactionnel est ensuite agité à température ambiante pendant 4 heures avant dilution au dichlorométhane. La phase organique est lavée avec une solution d'hydrogénocarbonate de sodium, séchée sur sulfate de magnésium. Après filtration, les solvants sont évaporés sous pression réduite. Le brut obtenu est purifié par chromatographie sur gel de silice (cyclohexane/acétate d'éthyle 95:5) ; l'intermédiaire **M** est isolé avec un rendement quantitatif (poudre blanche, 0.47 g).

### Synthèse de l'intermédiaire N :

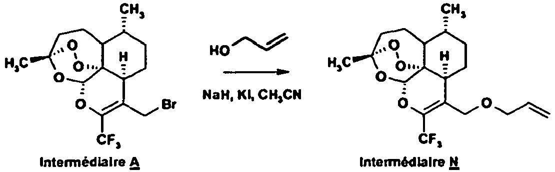

A une suspension dans l'acétonitrile (2 mL) de NaH (60% dans l'huile, 0.11 g, 2.72 mmol, 2.5 éq.) est ajouté l'alcool allylique (60 µL, 0.87 mmol) ; après 5 minutes d'agitation, est ajoutée à ce mélange, une solution dans l'acétonitrile (2 mL) de l'intermédiaire **A** (0.45 g, 1.1 mmol, 1.25 éq.) et d'iodure de potassium (en quantité catalytique). Le milieu réactionnel est agité à température ambiante pendant 16 heures avant dilution à l'acétate d'éthyle. La phase organique est lavée avec une solution d'hydrogénocarbonate de sodium, séchée sur sulfate de magnésium. Après filtration, les solvants sont évaporés sous pression réduite. Le brut obtenu est purifié par chromatographie sur gel de silice (cyclohexane/acétate d'éthyle 95:5 à 90:10) ; l'intermédiaire **N** est isolé avec un rendement de 34% (poudre blanche, 0.15 g).

### Couplage des intermédiaires M et N :

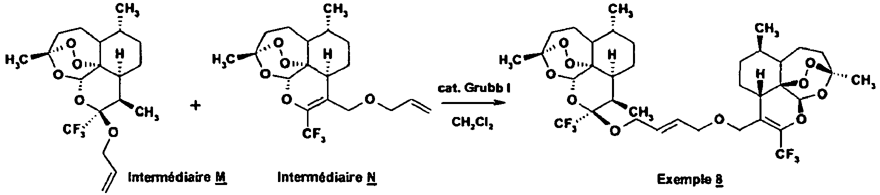

A une solution dans le dichlorométhane (3 mL) des intermédiaires **M** (0.147 g, 0.37 mmol, 1 éq.) et **N** (0.146 g, 0.37 mmol, 1 éq.) est ajouté le catalyseur de Grubb de 1^{ère} génération (cat. Grubb I) (0.031 g, 0.037 mmol, 0,1 éq.). Le mélange est agité à 40°C pendant 20 heures puis refroidi et filtré sur célite. Les solvants sont évaporés sous pression réduite. Le brut obtenu est purifié par chromatographie sur gel de silice (cyclohexane/acétate d'éthyle 95:5) ; le composé 8 est isolé avec un rendement de 15% (poudre blanche, 0.043 g).

### Synthèse du composé 9

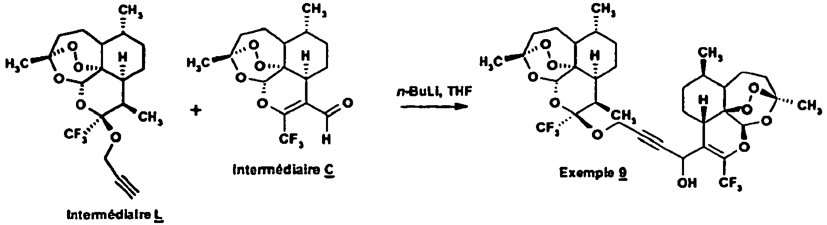

A une solution de l'intermédiaire **L** (0.047 g, 0.12 mmol) dans le tétrahydrofurane (1 mL) à -78°C est ajouté goutte à goutte le *n*-butyl-lithium (*n*-BuLi) (1.6 M dans l'hexane, 100 µL, 0.16 mmol, 1.3 éq.). Après 20 minutes d'agitation à -78°C, une solution de l'intermédiaire **C** (0.042 g, 0.12 mmol, 1 éq.) dans le tétrahydrofurane (1 mL) est additionnée. Le mélange réactionnel est ensuite remonté à température ambiante et agité 16 heures. Après addition d'une solution saturée de chlorure d'ammonium, le mélange est extrait au dichlorométhane. La phase organique est ensuite séchée sur sulfate de magnésium, filtrée et condensée sous pression réduite. Le brut obtenu est purifié par chromatographie sur gel de silice (cyclohexane/acétate d'éthyle 85:15) ; le composé 9 est isolé avec un rendement de 40% (poudre blanche, 0.035 g).

### Synthèse du composé 10

### Synthèse de l'intermédiaire O :

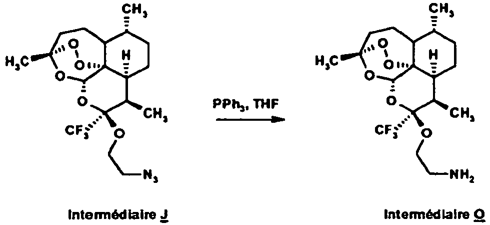

A une solution de l'intermédiaire **J** (0.19 g, 0.45 mol) dans le tétrahydrofurane (4 mL) est ajoutée la triphénylphosphine (PPh₃) (0.12 g, 0.45 mmol, 1 éq.). Après 24 heures à température ambiante, 2 mL d'eau sont ajoutés et l'agitation est maintenue 24 heures supplémentaires. Après évaporation des solvants sous pression réduite, le brut obtenu est purifié par chromatographie sur gel de silice (cyclohexane/acétate d'éthyle 100:0 à 94:6) ; l'intermédiaire **O** est isolé avec un rendement de 42% (poudre blanche, 0.075 g).

### Couplage des intermédiaires A et O :

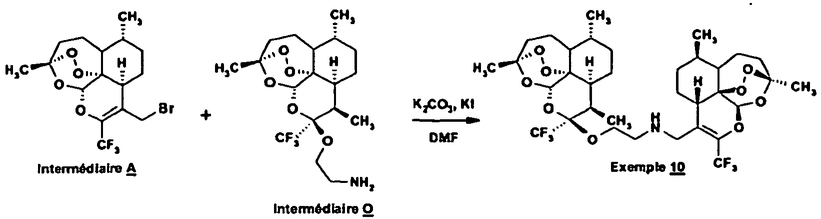

A une solution de l'intermédiaire **A** (0.070 g, 0.17 mmol) dans le diméthylformamide (DMF) (0.5 mL) sont ajoutés le carbonate de potassium (0.052 g, 0.37 mmol, 2.2 éq.) et l'iodure de potassium (en quantité catalytique). Après 10 minutes d'agitation, une solution de l'intermédiaire **O** dans le diméthylformamide (0.5 mL) est additionnée. Le mélange réactionnel est alors chauffé à 70°C pendant 2.5 heures. Après retour à température ambiante, de l'eau et de l'acétate d'éthyle sont ajoutés au milieu. La phase organique est lavée avec une solution saturée de chlorure de sodium et séchée sur sulfate de magnésium. Après évaporation des solvants sous pression réduite, le brut obtenu est purifié par chromatographie sur gel de silice (dichlorométhane/acétate d'éthyle 100:0 à 85:15) ; le composé **10** est isolé avec un rendement de 34% (poudre jaune pâle, 0.042 g).

### Synthèse du composé 11

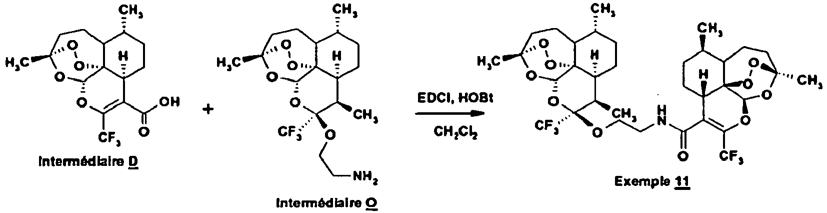

A une solution de l'intermédiaire **D** (0.11 g, 0.30 mmol) dans le dichlorométhane (5 mL) sont ajoutés du chlorhydrate de 1-(3-diméthylaminopropyl)-3-éthylcarbodiimide (EDCI) (0.17 g, 0.91 mmol, 3 éq.) et du 1-hydroxybenzotriazole (HOBt) (0.12 g, 0.91 mmol, 3 éq.). Après 30 minutes d'agitation à température ambiante, une solution de l'intermédiaire **O** (0.12 g, 0.30 mmol, 1 éq.) dans le dichlorométhane (5 mL) est additionnée. Le mélange réactionnel est agité 2 heures à température ambiante. Après addition d'eau, le mélange est extrait au dichlorométhane. La phase organique est lavée avec une solution saturée de chlorure de sodium puis séchée sur sulfate de magnésium. Après filtration, les solvants sont évaporés sous pression réduite. Le brut obtenu est purifié par chromatographie sur gel de silice (dichlorométhane/acétate d'éthyle 99:1 à 97:3) ; le composé **11** est isolé avec un rendement de 7% (poudre blanche, 0.016 g).

### Synthèse des composés 12 et 20

### Synthèse d l'intermédiaire P :

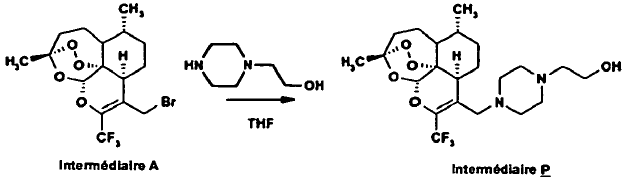

A une solution de l'intermédiaire **A** (1 g, 2.4 mmol) dans le tétrahydrofurane (15 mL) à 0°C est additionnée la *N*-(2-hydroxyéthyl)pipérazine (1.19 mL, 9.7 mmol, 4 éq.). Le milieu réactionnel est agité à 0°C pendant 4 heures. Après addition d'eau, le mélange est extrait à l'éther diéthylique. La phase organique est lavée avec une solution saturée de chlorure de sodium puis séchée sur sulfate de magnésium. Après filtration, les solvants sont évaporés sous pression réduite. Le brut obtenu est purifié par chromatographie sur gel de silice (dichlorométhane/méthanol 97:3 à 94:6) ; l'intermédiaire **P** est isolé avec un rendement de 84% (poudre jaune, 0.92 g).

### Synthèse de l'intermédiaire U :

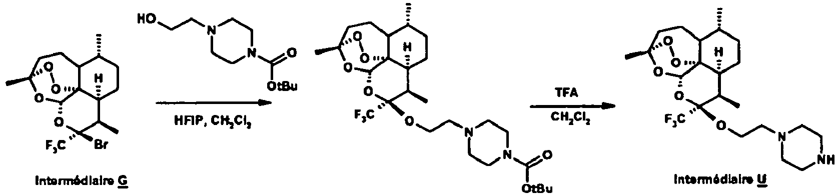

A une solution de l'intermédiaire G (0.60 g, 1.44 mmol) dans le dichlorométhane (6.0 mL) sont ajoutés successivement la *N*-Boc-4-(2-hydroxy-éthyl)-pipérazine (1.66 g, 7.22 mmol, 5 éq.) et l'hexafluoroisopropanol (HFIP) (0.76 mL, 7.22 mmol, 5 éq.). Le mélange réactionnel est agité à température ambiante pendant 18h. La phase organique est lavée avec une solution d'hydrogénocarbonate de sodium puis séchée sur sulfate de magnésium. Après filtration, les solvants sont évaporés sous pression réduite. Le brut obtenu est purifié par chromatographie sur gel de silice (cyclohexane/acétate d'éthyle 80:20). Le composé attendu, isolé avec un rendement de 22% (poudre blanche, 0.18 g), est mis en solution dans le dichlorométhane (2.0 mL) à laquelle est ajouté de l'acide trifluoroacétique (TFA) (0.75mL). Le mélange réactionnel est agité pendant 30 minutes à température ambiante puis concentré sous pression réduite. Le résidu est dilué dans du dichlorométhane. La phase organique est successivement lavée avec une solution saturée d'hydrogénocarbonate de sodium, et une solution saturée de chlorure de sodium, puis séchée sur sulfate de sodium. Après filtration, les solvants sont évaporés sous pression réduite. Le brut obtenu est purifié par chromatographie sur gel de silice (dichlorométhane/méthanol/ammoniaque aqueux 95:5:0.5); l'intermédiaire **U** est isolé avec un rendement de 61 % (poudre blanche, 0.095 g).

### Couplage des intermédiaires G et P :

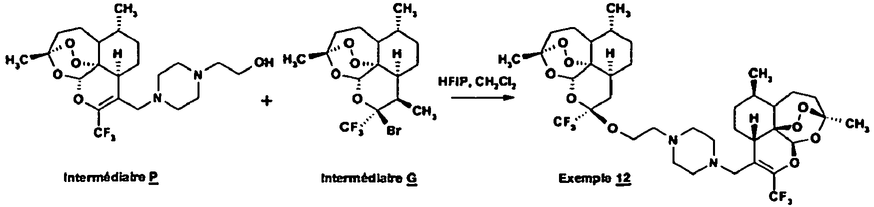

A une solution de l'intermédiaire **G** (0.1 g, 0.24 mmol) dans le dichlorométhane (1.5 mL) sont ajoutés successivement l'intermédiaire **P** (0.22 g, 0.48 mmol, 2 éq.) et l'hexafluoroisopropanol (127 µL, 1.2 mmol, 5 éq.). Le mélange réactionnel est ensuite agité à température ambiante pendant 2 heures avant dilution au dichlorométhane. La phase organique est lavée avec une solution d'hydrogénocarbonate de sodium puis séchée sur sulfate de magnésium. Après filtration, les solvants sont évaporés sous pression réduite. Le brut obtenu est purifié par chromatographie sur gel de silice (cyclohexane/acétate d'éthyle 95:5 à 80:20); le composé **12** est isolé avec un rendement de 6% (poudre jaune pâle, 0.022 g).

### Couplage des intermédiaires A₁ et U :

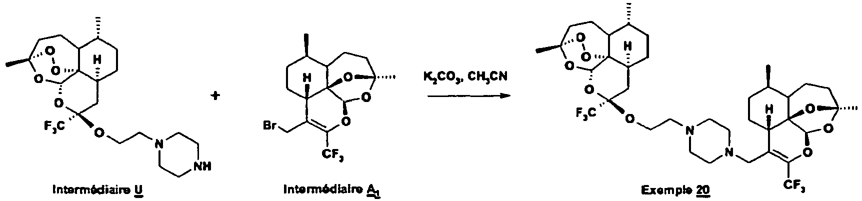

A une solution de l'intermédiaire **U** (0.35 g, 0.76 mmol) dans l'acétonitrile (5.0 mL) sont ajoutés du carbonate de potassium (0.21 g, 1.51 mmol, 2 éq.) et l'intermédiaire **A₁** (0.30 g, 0.76 mmol, 1 éq.). Le mélange réactionnel est agité pendant 2 heures à température ambiante avant dilution dans l'eau et l'acétate d'éthyle. La phase aqueuse est extraite à l'acétate d'éthyle. Les phases organiques combinées sont lavées avec une solution saturée de chlorure de sodium, puis séchées sur sulfate de sodium. Après filtration, les solvants sont évaporés sous pression réduite. Le brut obtenu est purifié par chromatographie sur gel de silice (dichlorométhane/acétate d'éthyle 97.5:2.5 à 92:8) ; le composé **20** est isolé avec un rendement de 88% (mousse blanche, 0.52 g).

### Synthèse du composé 13

### Synthèse de l'intermédiaire Q :

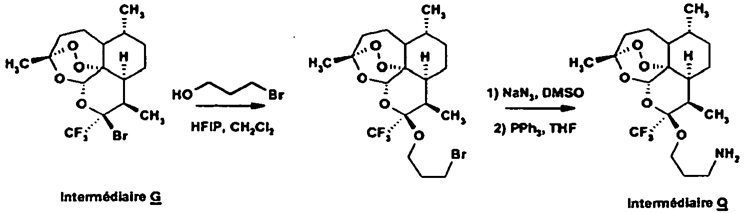

Etape 1 : A une solution de l'intermédiaire **G** (1.0 g, 2.41 mmol) dans le dichlorométhane (14 mL) sont ajouté successivement le 2-bromo-propanol (2.77 mL, 24.1 mmol, 10 éq.) et l'hexafluoroisopropanol (HFIP)(1.27 mL, 12.0 mmol, 5 éq.). Le mélange réactionnel est ensuite agité à température ambiante pendant 2.5 heures avant dilution au dichlorométhane. La phase organique est lavée avec une solution d'hydrogénocarbonate de sodium puis séchée sur sulfate de magnésium. Après filtration, les solvants sont évaporés sous pression réduite. Le brut obtenu est purifié par chromatographie sur gel de silice (cyclohexane/acétate d'éthyle 95:5) ; l'intermédiaire bromé est isolé avec un rendement de 55% (huile claire, 0.63 g).

Etape 2: A une solution de l'intermédiaire bromé (0.63 g, 1.38 mmol) dans le diméthylsulfoxyde (12 mL) est ajouté de l'azoture de sodium (0.18 g, 2.75 mmol, 2 éq.). Le mélange réactionnel est agité à température ambiante pendant 2.5 heures. Après dilution à l'acétate d'éthyle, la phase organique est lavée à l'eau puis séchée sur sulfate de magnésium. Après filtration, les solvants sont évaporés sous pression réduite et l'intermédiaire azoture, obtenu quantitativement, est engagé directement dans l'étape suivante (poudre blanche).

Etape 3: A une solution de l'intermédiaire azoture (0.60 g, 1.38 mmol) dans le tétrahydrofurane (6 mL) est ajoutée la triphénylphosphine (0.36 g, 1.38 mmol, 1 éq.). Après 24 heures à température ambiante, 2 mL d'eau sont ajoutés et l'agitation est maintenue 24 heures supplémentaires. Après évaporation des solvants sous pression réduite, le brut obtenu est purifié par chromatographie sur gel de silice (cyclohexane/acétate d'éthyle 100:0 à 85:15 puis dichlorométhane/méthanol 99:1 à 70:30); l'intermédiaire **Q** est isolé avec un rendement de 62% (poudre blanche, 0.35 g).

### Couplage des intermédiaires D et Q :

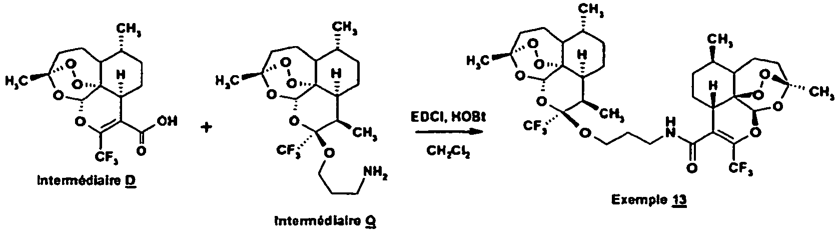

A une solution de l'intermédiaire **D** (0.27 g, 0.74 mmol) dans le dichlorométhane (10 mL) sont ajoutés EDCI (0.43 g, 2.24 mmol, 3 éq.) et HOBt (0.30 g, 2.24 mmol, 3 éq.). Après 30 minutes d'agitation à température ambiante, une solution de l'intermédiaire **Q** (0.30 g, 0.74 mmol, 1 éq.) dans le dichlorométhane (10 mL) est ajoutée. Le mélange réactionnel est agité 2 heures à température ambiante. Après addition d'eau, le mélange est extrait au dichlorométhane. La phase organique est lavée avec une solution saturée de chlorure de sodium puis séchée sur sulfate de magnésium. Après filtration, les solvants sont évaporés sous pression réduite. Le brut obtenu est purifié par chromatographie sur gel de silice (cyclohexane/acétate d'éthyle 90:10 à 80:20) ; le composé **13** est isolé avec un rendement de 59% (poudre blanche, 0.335 g).

### Synthèse du composé 14

### Synthèse de l'intermédiaire S :

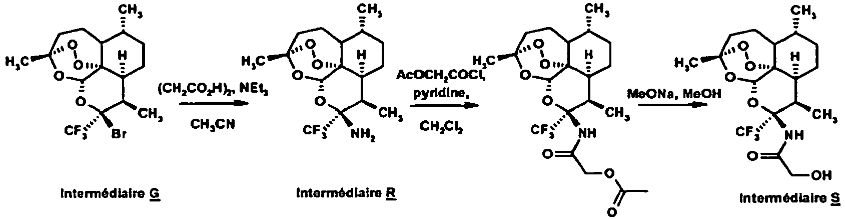

Etape 1 : A une solution de l'intermédiaire **G** (1.0 g, 2.41 mmol) dans l'acétonitrile (10 mL) sont additionnés l'acide succinique (2.84 g, 24.1 mmol, 10 éq.) et la triéthylamine (1.69 mL, 12.0 mmol, 5 éq.). Le mélange réactionnel est agité 96 heures à température ambiante. Une solution d'hydrogénocarbonate de sodium est ajoutée et l'extraction réalisée à l'acétate d'éthyle. La phase organique est ensuite lavée avec une solution saturée de chlorure de sodium, séchée sur sulfate de magnésium, filtrée puis condensée sous pression réduite. Le brut obtenu est purifié par chromatographie sur gel de silice (cyclohexane/acétate d'éthyle 95:5 à 90:10) ; l'intermédiaire **R** est isolé avec un rendement de 33% (poudre beige, 0.28 g).

Etape 2 : A une solution de l'intermédiaire **R** (0.1 g, 0.28 mmol) dans le dichlorométhane (2.5 mL) sont ajoutés le chlorure d'acétoxyacétyle (40 µL, 0.37 mmol, 1.3 éq.) et la pyridine (30 µL, 0.37 mmol, 1.3 éq.). Après 24 heures à température ambiante, une nouvelle addition de chlorure d'acyle (1.3 éq.) et de pyridine (1.3 éq.) est réalisée et le milieu réactionnel est agité 6 heures supplémentaires. Une solution saturée de chlorure d'ammonium est ajoutée et le mélange extrait au dichlorométhane. La phase organique est séchée sur sulfate de magnésium, filtrée puis condensée sous pression réduite. Le brut obtenu est purifié par chromatographie sur gel de silice (dichlorométhane/acétate d'éthyle 95:5 à 92.5:7.5) ; l'intermédiaire acétoxy est isolé avec un rendement de 44% (poudre blanche, 0.055 g).

Etape 3 : A une solution de l'intermédiaire acétoxy (0.053 g, 0.117 mmol) dans le méthanol (2 mL) à 0°C est additionnée une solution de méthanolate de sodium (0.010 g, 0.176 mmol) dans le méthanol (0.5 mL). Après 1 heure d'agitation à 0°C, le milieu est dilué avec de l'acétate d'éthyle et de l'eau. La phase organique est ensuite séchée sur sulfate de magnésium, filtrée puis condensée sous pression réduite. Le brut obtenu est purifié par chromatographie sur gel de silice (dichlorométhane/acétate d'éthyle 90:10 à 85:15) ; l'intermédiaire **S** est isolé avec un rendement de 94% (huile incolore, 0.045 g).

### Couplage des intermédiaires A et S :

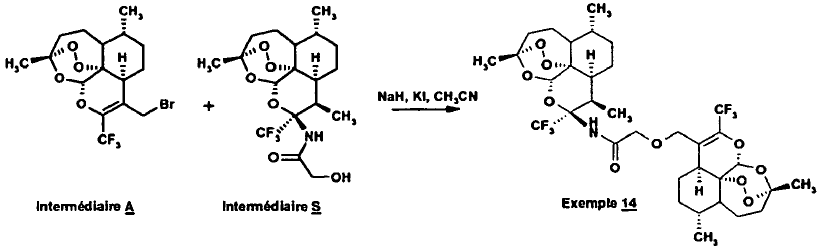

A une solution de l'intermédiaire **S** (0.04 g, 0.1 mmol) dans l'acétonitrile (2 mL) est additionné l'hydrure de sodium (60% dans l'huile, 0.01 g, 0.25 mmol, 2.5 éq.). Après 5 minutes d'agitation, une solution de l'intermédiaire **A** (0.05 g, 0.12 mmol, 1.2 éq.) et d'iodure de potassium (en quantité catalytique) dans l'acétonitrile (1 mL) est ajoutée. Le mélange réactionnel est agité à température ambiante pendant 30 heures (additions renouvelées d'hydrure de sodium (2.5 éq.)). Après addition d'une solution saturée d'hydrogénocarbonate de sodium, l'extraction est réalisée à l'acétate d'éthyle. La phase organique est ensuite séchée sur sulfate de magnésium, filtrée et condensée sous pression réduite. Le brut obtenu est purifié par chromatographie sur gel de silice

(cyclohexane/acétate d'éthyle 85:15 à 75:25) ; le composé **14** est isolé avec un rendement de 21% (poudre blanche, 0.016 g).

### Synthèse du composé 15

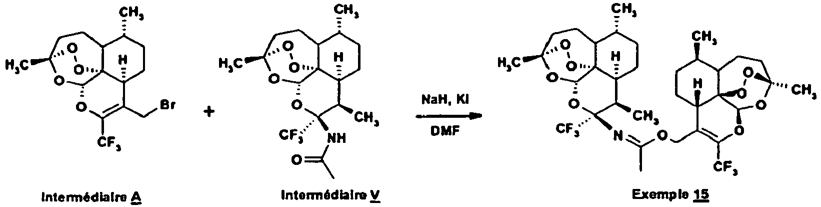

### Synthèse de l'intermédiaire V :

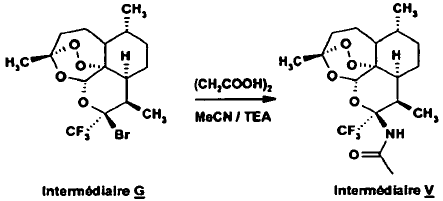

A une solution de l'intermédiaire **G** (1.47 g, 3.5 mmol, 1 éq.) dans l'acétonitrile sont ajoutés successivement et à température ambiante, l'acide succinique (4.19 g, 35.5 mmol, 10 éq.) et la triéthylamine (1.80 g, 17.7 mmol, 5 éq.). Après 3 jours d'agitation à température ambiante, le milieu réactionnel est dilué avec de l'acétate d'éthyle (60 mL) et lavé avec une solution saturée de NaHCO₃ (30 mL). La phase organique est séchée sur MgSO₄, filtrée puis concentrée sous pression réduite. Le brut réactionnel est purifié par chromatographie sur colonne gel de silice avec un gradient d'éluant (cyclohexane/acétate d'éthyle: 100 à 40/60). L'intermédiaire **V** est obtenu avec un rendement de 30 % (401 mg).

### Couplage des intermédiaires A et V :

A une solution de l'intermédiaire **V** (0.1 g, 0.25 mmol) dans le diméthylformamide (1.2 mL) à 0°C, est additionné l'hydrure de sodium (60% dans l'huile, 0.02 g, 0.51 mmol, 2 éq.). Après 5 minutes d'agitation, l'intermédiaire **A** (0.21 g, 0.51 mmol, 2 éq.) et d'iodure de potassium (0.01 g, 0.05 mmol, 0.2 éq.) sont ajoutés. Le mélange réactionnel est agité à température ambiante pendant 2 heures. Après hydrolyse avec une solution saturée de chlorure de sodium, le mélange réactionnel est extrait au diéthyl éther puis la phase organique est séchée sur sulfate de magnésium, filtrée et condensée sous pression réduite. Le brut obtenu est purifié par chromatographie sur gel de silice (éther de pétrole/diéthyl éther 98:2 à 90: 10) ; le composé **15** est isolé avec un rendement de 40% (poudre blanche, 0.073 g).

### Synthèse du composé 21

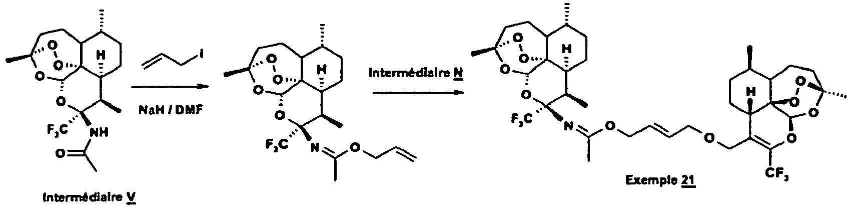

Etape 1 : A une solution de l'intermédiaire **V** (250 mg, 0.635 mmol) dans le diméthylformamide (DMF) anhydre (4 mL) est ajouté à 0 °C l'hydrure de sodium (76 mg, 1.9 mmol, 3 éq.) puis le iodure allylique (174 µL, 1.9 mmol, 3 éq.). Le milieu réactionnel est laissé revenir à température ambiante. Après 4h30 d'agitation, il est hydrolysé avec une solution de NaCl saturée. La phase organique est séchée sur MgSO₄, filtrée puis évaporée sous pression réduite. Le *O*-allyl iminoéther (277 mg, 80%) est obtenu après purification par chromatographie sur gel de silice avec un gradient d'éluant (éther de pétrole/éther diéthylique : 98/2 à 95/5).

Etape 2 : A une solution de l'intermédiaire N (131 mg, 0.335 mmol, 0.8 éq.) dans le dichlorométhane (1.6 mL) est ajouté le *O*-allyl iminoéther (174 mg, 0.401 mmol, 1 éq.) isolé ci-dessus, puis le catalyseur de Grubb de 1ère génération (33 mg, 0.043 mmol, 0.1 éq.). Après 23h d'agitation à température ambiante, le milieu réactionnel est filtré sur de la silice puis est concentré sous pression réduite. Le composé **21** (47 mg, 15 %) est obtenu après purification par chromatographie sur gel de silice avec un gradient d'éluant (cyclohexane/acétate d'éthyle : 100 à 85/15).

### Synthèse des composés 22, 23 et 24

### Synthèse de l'intermédiaire Wa :

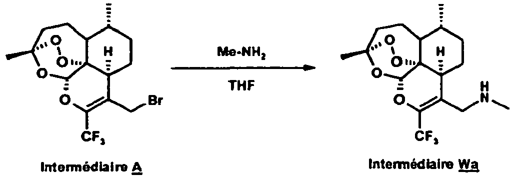

Une solution de l'intermédiaire **A** (100 mg, 0.241 mmol) dans le THF anhydre (1.2 mL) est ajoutée pendant 45 minutes, à 0 °C et sous argon, à une solution de méthylamine [8.03 M dans l'éthanol (300 µL, 2.41 mmol, 10 éq.)] dans du tétrahydrofurane (THF) anhydre (12.5 mL). Après 2h d'agitation à température ambiante, la réaction est diluée dans de l'éther diéthylique et lavée avec une solution de NaCl saturée. La phase organique est séchée sur MgSO₄, filtrée et concentrée sous pression réduite. Le brut réactionnel est purifié par chromatographie sur gel de silice avec un gradient d'éluant (cyclohexane/acétate d'éthyle : 80/20 à 10/90). L'intermédiaire **Wa** est obtenu avec 85 % de rendement (75 mg).

### Synthèse de l'intermédiaire Wb :

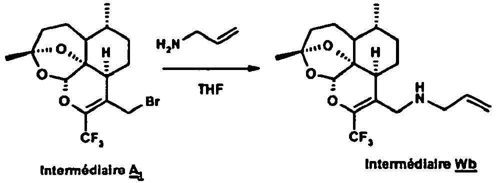

L'intermédiaire **Wb** est obtenu suivant le même procédé avec un rendement de 47 % par réaction de l'intermédiaire **A₁** avec l'allylamine.

### Synthèse de l'intermédiaire Xa :

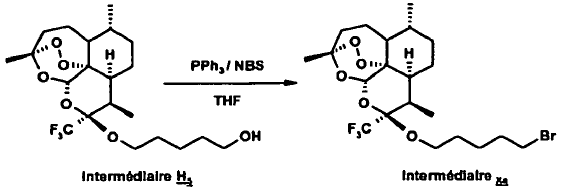

A une solution de l'intermédiaire **H₅** (110 mg, 0.250 mmol) dans le THF anhydre (1 mL), à - 15 °C et sous argon, est ajouté la triphénylphosphine (78 mg, 0.301 mmol, 1.2 éq.) puis le *N*-bromosuccinimide (NBS) (67 mg, 0.376 mmol, 1.5 éq.). Le milieu réactionnel est laissé remonter à température ambiante. Après 1h d'agitation supplémentaire, la réaction est diluée dans de l'éther diéthylique et lavée avec une solution de NaCl saturée. La phase organique est séchée sur MgSO₄, filtrée et concentrée sous pression réduite. Le brut réactionnel est purifié par chromatographie sur gel de silice avec un gradient d'éluant (cyclohexane/acétate d'éthyle 100 à 95:5). L'intermédiaire **Xa** est obtenu avec 75% de rendement (92 mg).

### Synthèse de l'intermédiaire Xb :

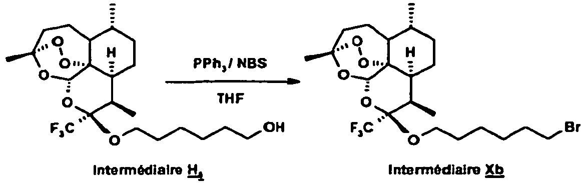

L'intermédiaire **Xb** est obtenu suivant le même procédé avec un rendement de 89% à partir de l'intermédiaire **H₆.**

### Synthèse de l'intermédiaire Xc :

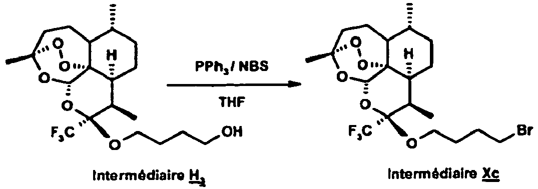

L'intermédiaire **Xc** est obtenu suivant le même procédé avec un rendement de 86% à partir de l'intermédiaire **H₃**.

### Couplage des intermédiaires W et X :

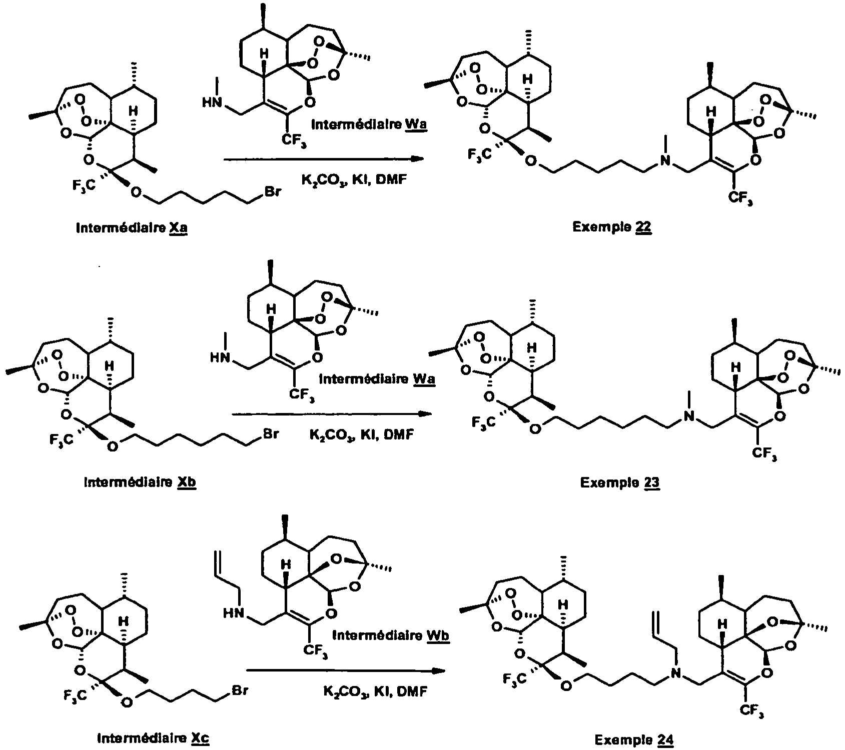

A une solution de l'intermédiaire **Xa** (93 mg, 0.186 mmol) dans le DMF anhydre (1.6 mL) est ajouté, à température ambiante et sous argon, l'iodure de potassium (34 mg, 0.204 mmol, 1.1 éq.), le carbonate de potassium (57 mg, 0.411 mmol, 2.2 éq.) puis l'intermédiaire **Wa** (74 mg, 0.203 mmol, 1.0 éq.) en solution dans le DMF anhydre (1.0 mL). Le milieu réactionnel est chauffé à 75 °C pendant 1h30. Une fois refroidi, le mélange réactionnel est dilué avec du dichlorométhane (5mL), lavé avec une solution de NaHCO₃ saturée (5 mL) puis avec une solution de NaCl saturée (5 mL). La phase organique est séchée sur MgSO₄, filtrée puis évaporée sous pression réduite. Le brut réactionnel est purifié par chromatographie sur gel de silice avec un gradient d'éluant (cyclohexane/acétate d'éthyle: 100 à 92/8). Le composé **22** est obtenu avec 70 % de rendement (105 mg).

Le composé **23** est obtenu avec un rendement de 18% en suivant le même mode opératoire, par couplage de l'intermédiaire **Wa** avec l'intermédiaire **Xb.**

Le composé **24** est obtenu avec un rendement de 76% en suivant le même mode opératoire, par couplage de l'intermédiaire **Wb** avec l'intermédiaire **Xc**.

### Synthèse du composé 25

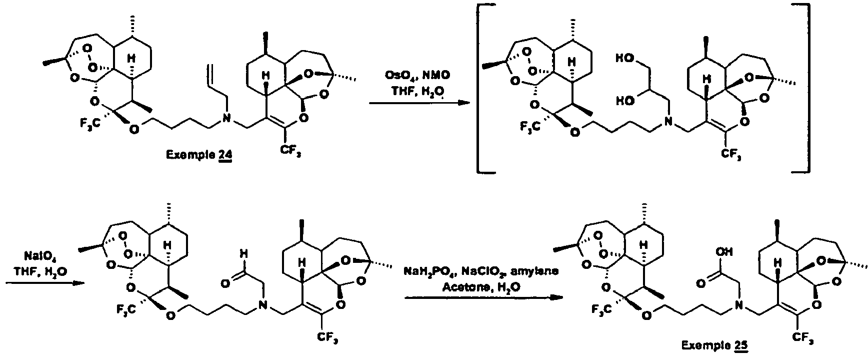

Etap e 1 : A une solution du composé **24** (0.4 g, 0.51 mmol) dans du tétrahydrofurane (20.0 mL) et de l'eau (5.0 mL) sont ajoutés OsO₄ (0.98 mL, 0.15 mmol, 0.3 éq.) et oxyde de N-méthylmorpholine (NMO) (78.1 mg, 0.67 mmol, 1.3 éq.). Le mélange réactionnel est agité à température ambiante pendant 18h. De l'eau (15.0 mL) et NaIO₄ (0.55 g, 2.56 mmol, 10 éq.) sont ajoutés, et le mélange réactionnel est agité pendant 1 heure à température ambiante, avant d'être diluée par ajout d'eau et d'acétate d'éthyle. La phase organique est lavée avec une solution saturée de thiosulfate de sodium, une solution saturée de chlorure de sodium, puis est séchée sur sulfate de magnésium. Après filtration, les solvants sont évaporés sous pression réduite.

Etape 2 : A une solution de l'aldéhyde brut précédemment formé (0.44 g, 0.57 mmol) dans l'acétone (15.0 mL) et l'eau (8.0 mL) sont ajoutés du phosphate de sodium (0.23 g, 1.70 mmol, 3 éq.), de l'amylène (0.27 mL, 2.55 mmol, 4.5 éq.) et du chlorite de sodium (0.15 g, 1.70 mmol, 3 éq.). Le mélange réactionnel est agité à température ambiante pendant 18 heures avant d'être diluée dans l'eau et l'acétate d'éthyle. La phase aqueuse est extraite à l'acétate d'éthyle. Les phases organiques combinées sont lavées à l'eau et avec une solution saturée de chlorure de sodium, puis séchée sur sulfate de magnésium. Après filtration, les solvants sont évaporés sous pression réduite. Le brut réactionnel est purifié par chromatographie sur gel de silice (acétate d'éthyle/méthanol 50:50). Le composé **25** est obtenu avec un rendement de 18% (gomme beige, 0.082 g).

### Synthèse du composé 26

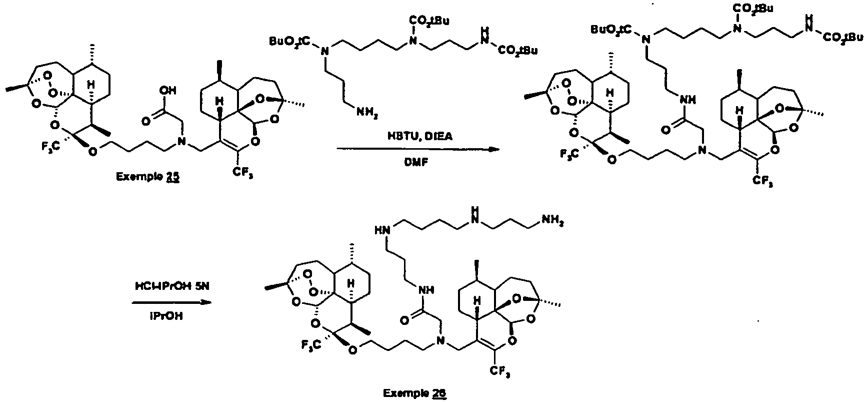

Etape 1 : A une solution de **26** (0.07 g, 0.089 mmol) dans du *N,N*-diméthylformamide (1.0 mL) sont ajoutés la polyamine (0.045 g, 0.089 mmol, 1 éq.), le 2-(1H-benzotriazole-1-yl)-1,1,3,3-tetraméthyluronium hexafluorophosphate (HBTU) (0.044 g, 0.12 mmol, 1.3 éq.) et de la diisopropyléthylamine (DIEA) (0.04 mL, 0.22 mmol, 2.5 éq.). Le mélange réactionnel est agité pendant 6 heures à température ambiante avant d'être diluée dans l'eau et l'acétate d'éthyle. La phase aqueuse est extraite à l'acétate d'éthyle. Les phases organiques combinées sont lavées à l'eau et avec une solution saturée de chlorure de sodium, puis séchée sur sulfate de magnésium. Après filtration, les solvants sont évaporés sous pression réduite. Le brut réactionnel est purifié par chromatographie sur gel de silice (dichlorométhane/acétate d'éthyle 90:10 à 70:30) ; le produit attendu est obtenu avec un rendement de 44% (0.050 g).

Etape 2 : A une solution de l'intermédiaire obtenu précédemment (0.05 g, 0.039 mmol) dans l'isopropanol (3.0 mL) est ajouté une solution d'acide chlorhydrique 5N dans l'isopropanol (1.0 mL). Le mélange réactionnel est agité à température ambiante pendant 18 heures puis est concentré sous pression réduite. **26** est obtenu avec un rendement de 39% sous forme de chlorhydrate (solide blanc, 0.015 g).

### Synthèse des composés 27 et 28

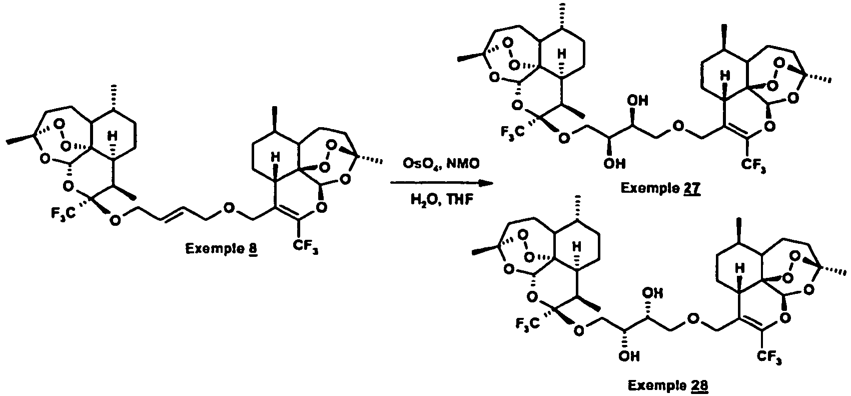

A une solution du composé **8** (0.11 g, 0.14 mmol) dans du tétrahydrofurane (3.5 mL) et de l'eau (0.9 mL) sont ajoutés OsO₄ (0.36 mL, 0.042 mmol, 0.3 éq.) et NMO (25 mg, 0.2 mmol, 1.5 éq.). Le mélange réactionnel est agité à température ambiante pendant 18h. La phase organique est lavée avec une solution saturée de thiosulfate de sodium, une solution saturée de chlorure de sodium, puis est séchée sur sulfate de magnésium. Après filtration, les solvants sont évaporés sous pression réduite. Le mélange brut est purifié par chromatographie sur gel de silice (cyclohexane/acétate d'éthyle 100:0 à 70:30). **27** est obtenu avec un rendement de 36% (mousse beige, 0.041 g). **28** est obtenu avec un rendement de 13% (mousse beige, 0.015 g).

### Synthèse du composé 29

### Synthèse de l'intermédiaire Y

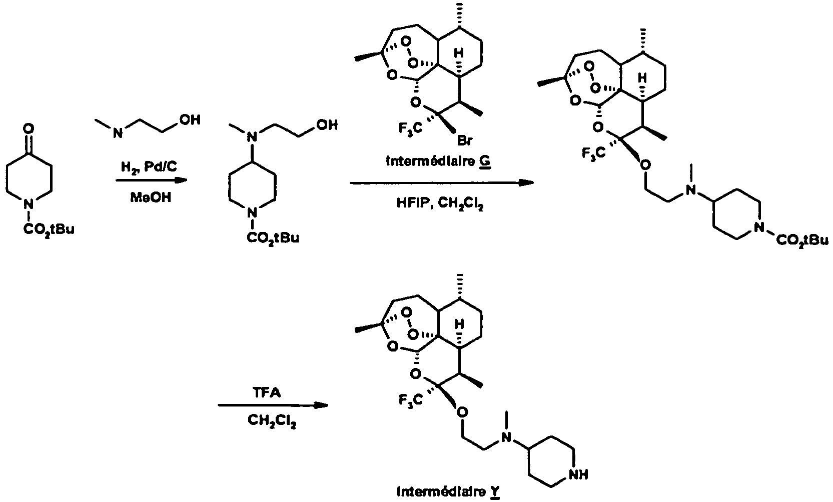

Etape 1 : A une solution de *N*-Boc-pipéridone (5.0 g, 0.025 mol) dans du méthanol (85 mL) sont ajoutés du 2-*N*-méthylaminoéthanol (7.0 mL, 0.087 mol, 3.5 éq.) et Pd/C (0.7 g). Le mélange réactionnel est agité à température ambiante sous atmosphère d'hydrogène pendant 18h. Le mélange est alors filtré sur célite, et les solvants sont évaporés sous pression réduite. Le brut est alors purifié par chromatographie sur gel de silice (dichlorométhane/méthanol 85:15). L'intermédiaire attendu est obtenu avec 97% de rendement (huile jaune, 6.4 g).

Etape 2: Une solution de l'intermédiaire **G** (1.0 g, 2.42 mmol), de l'intermédiaire précédemment formé (6.4 g, 25.6 mmol) et d'hexafluoroisopropanol (HFIP) (1.9 mL, 18.1 mmol) est agitée à température ambiante pendant 14 jours. Les solvants sont alors évaporés sous pression réduite, et le brut réactionnel est purifié par chromatographie sur gel de silice (dichlorométhane/méthanol 100:0 à 97:3). L'intermédiaire attendu est obtenu avec un rendement de 15% (0.21 g).

Etape 3 : A une solution de l'intermédiaire précédemment formé (0.21 g, 0.36 mmol) dans le dichlorométhane (2.0 mL) est additionné de l'acide trifluoroacétique (TFA) (0.6 mL). Le mélange réactionnel est agité à température ambiante pendant 3 heures, puis basifié par ajout d'une solution aqueuse de carbonate de potassium. La phase aqueuse est extraite avec du dichlorométhane. Les phases organiques combinées sont séchées sur du sulfate de magnésium. Après filtration, le solvant est évaporé sous pression réduite. Le brut est purifié par chromatographie sur gel de silice (dichlorométhane/méthanol/ammoniaque aqueux 80:20:1 à 70:30:1) ; l'intermédiaire **Y** est obtenu avec un rendement de 42% (huile jaune, 0.074 g).

### Couplage des intermédiaires A et Y

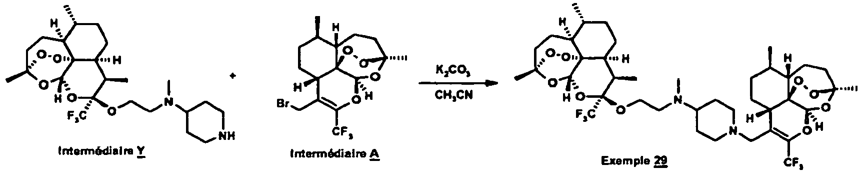

A une solution d'intermédiaire **Y** (0.074 g, 0.15 mmol) dans l'acétonitrile (1.0 mL) sont ajoutés du carbonate de potassium (0.023 g, 0.16 mmol, 1.1 éq.) et l'intermédiaire **A** (0.068 g, 0.16 mmol, 1.1 éq.). Le mélange réactionnel est agité et chauffé à 90°C pendant 18h. Le mélange est alors refroidit à température ambiante et dilué dans de l'eau et de l'acétate d'éthyle. La phase aqueuse est extraite à l'acétate d'éthyle. Les phases organiques combinées sont lavées à l'eau et avec une solution saturée de chlorure de sodium, puis séchée sur sulfate de magnésium. Après filtration, les solvants sont évaporés sous pressions réduite. Le brut réactionnel est purifié par chromatographie sur gel de silice (cyclohexane/acétate d'éthyle 60:40 à 40:60) ; le composé **29** est obtenu avec un rendement de 7% (solide blanc, 0.008 g).

### EXEMPLE 2 : Activité cytotoxique des composés de l'invention

L'activité cytotoxique des composés selon l'invention a été évaluée en mesurant l'inhibition de la prolifération cellulaire de lignées tumorales d'origine humaine, telle que la lignée A549 (poumon) et la lignée Namalwa (lymphome). Cette activité est exprimée par l'IC₅₀, concentration de produit testé capable d'inhiber à 50% la prolifération cellulaire. La méthode employée est la mesure par luminescence de l'ATP résiduel après 72 heures d'incubation en utilisant le kit « ATPLite^{®} » commercialisé par Perkin Elmer, comme décrit dans la publication suivante : « Measurement of cytotoxicity by ATP - based luminescence assay in primary cell cultures and cell lines ». I.A. Cree, P.E. Andreotti, Toxicology in Vitro, 11, 553-6, (1997).

A titre d'exemple, les propriétés cytotoxiques de quelques composés de l'invention évalués sur les lignées A549 et Namalwa, sont reportées dans le tableau suivant :

| **Produit** | **IC₅₀ (exprimée en nM)** | |
|---|---|---|
| | **A549** | **Namalwa** |
| **1** | 5.2 | 6.8 |
| **2** | 25 | 27 |
| **3** | 6.7 | 5.5 |
| **4** | 27 | 19 |
| **7** | 45 | 30 |
| **8** | 27 | 13 |
| **10** | 27 | 19 |
| **16** | 19 | 42 |
| **17** | 62 | 130 |
| **21** | 30 | 35 |
| **22** | 46 | 83 |
| **28** | 22 | 21 |

Compte-tenu de ces propriétés cytotoxiques, les composés de l'invention peuvent être utilisés en thérapeutique humaine dans le traitement de la pathologie cancéreuse. Les préparations pharmaceutiques contenant ces principes actifs peuvent être mises en forme pour l'administration notamment par voie orale, intraveineuse ou sous-cutanée.

## Revendications

1. Dérivé dimérique de l'artémisinine 10-trifluorométhylée de formule (I) : 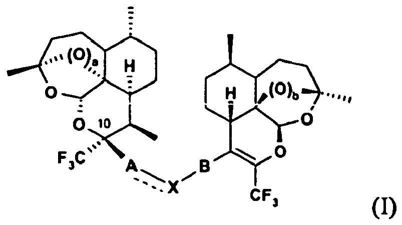 ou un sel pharmaceutiquement acceptable de celui-ci,
pour lequel :
- a et b représentent, indépendamment l'un de l'autre, 1 ou 2 mais ne peuvent représenter en même temps 1,
- A représente :
o un hétéroatome choisi parmi un atome d'azote, d'oxygène et de soufre, l'atome d'azote étant éventuellement substitué par un radical R1 choisi parmi un atome d'hydrogène, un groupement (C₁-C₆)alkyle, (C₂-C₆)alcényle, (C₂-C₆)alcynyle, (C₃-C₈)cycloalkyle, aryl-(C₁-C₆)alkyle, hétéroaryl-(C₁-C₆)alkyle, hétérocycle-(C₁-C₆)alkyle, aryle éventuellement substitué, hétéroaryle éventuellement substitué, -COR2, -CO₂R2, C(O)NR2R2bis, -SO₂R2,-CH₂C(O)OR2 et -CH₂C(O)NR2R2bis,
avec R2 représentant un atome d'hydrogène ou un groupement (C₁-C₆)alkyle, (C₂-C₆)alcényle, (C₂-C₆)alcynyle, polyamino éventuellement substitué, (C₃-C₈)cycloalkyle, aryl-(C₁-C₆)alkyle, hétéroaryl-(C₁-C₆)alkyle, aryle éventuellement substitué ou hétéroaryle éventuellement substitué, et R2bis représentant un atome d'hydrogène ou un groupement (C₁-C₆)alkyle, ou
o un hétérocycle saturé comprenant un ou plusieurs hétéroatomes choisis parmi les atomes d'oxygène, de soufre et d'azote, dont au moins un atome d'azote lié au carbone 10,
- 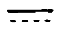 représente une liaison simple lorsque A représente un atome d'oxygène ou de soufre ou un hétérocycle, ou représente une liaison simple ou une liaison double lorsque A représente un atome d'azote, ledit atome d'azote étant substitué par un radical R1 tel que défini ci-dessus lorsque représente une liaison simple,
- B représente un groupe -CH₂-Y-, -C(=O)-Y- ou -CH(OR3)-,
avec Y représentant O, S, N-R1 ou un hétérocycle, R1 étant tel que défini ci-dessus, et
R3 représentant un atome d'hydrogène ou un groupement (C₁-C₆)alkyle ou aryle, et
- X représente :
■ lorsque représente une liaison double :
un groupement =C(X1)-(O-X2)_{c}- avec X1 et X2 représentant, indépendamment l'un de l'autre, un groupement (C₁-C₆)alkyle ou (C₂-C₆)alcényle et c représentant 0 ou 1, ou
■ lorsque représente une liaison simple :
o un groupement (C₁-C₆)alkyle éventuellement substitué par un ou plusieurs groupes OH; (C₂-C₆)alcényle; (C₂-C₆)alcynyle; [(C₁-C₆)alkyl]ₙ-(C₃-C₈)cycloalkyl-[(C₁-C₆)alkyle]ₚ ; [(C₁-C₆)alkyl]ₙ-hétérocycle-[(C₁-C₆)alkyle]ₚ ; [(C₁-C₆)alkyl]ₙ-aryl-[(C₁-C₆)alkyle]ₚ ; [(C₁-C₆)alkyl]ₙ-hétéroaryl-[(C₁-C₆)alkyle]ₚ ; avec n et p représentant, indépendamment l'un de l'autre, 0 ou 1,
o un groupement -CO-(CH₂)_{q}- ou -CO-(CH₂)_{q}-CO- pour lequel q représente un nombre entier égal à 1, 2, 3 ou 4, ou
o un groupement -COᵣ-(CH₂)ₛ-Z-(CH₂)ₜ-COᵤ- pour lequel r et u représentent, indépendamment l'un de l'autre, un nombre entier égal à 0 ou 1,
s et t représentent, indépendamment l'un de l'autre, un nombre entier égal à 0, 1, 2, 3 ou 4, et
Z représente un groupe -S-, -S-S-, -SO-, -SO₂-, -Se-Se-, -O-P(O)(OR3)-O-, -NR1-, (C₃-C₈)-cycloalkyle, aryle ou hétéroaryle, avec R1 et R3 tels définis ci-dessus.

2. Dérivé dimérique selon la revendication 1, **caractérisé en ce que** A représente un atome d'oxygène ou d'azote.

3. Dérivé dimérique selon l'une quelconque des revendications 1 et 2, **caractérisé en ce que** B représente un groupement -CH₂O-, -CH₂NR1-, -C(=O)NR1-, -CH(OR3)- et - CH₂-(hétérocycle)-, avec R1 et R3 tels que défini à la revendication 1.

4. Dérivé dimérique selon l'une quelconque des revendications 1 à 3, **caractérisé en ce que** 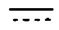 représente une liaison simple et X représente un groupement (C₁-C₆)alkyle, (C₂-C₆)alcényle, (C₂-C₆)alcynyle, (C₁-C₆)akyl-hétéroaryl-(C₁-C₆)akyle, -(CH₂)_{q}-NR1- ou - CO-(CH₂)_{q}-, avec q tel que défini à la revendication 1.

5. Dérivé dimérique selon l'une quelconque des revendications 1 à 4, **caractérisé en ce qu'**il est choisi parmi les composés suivants :

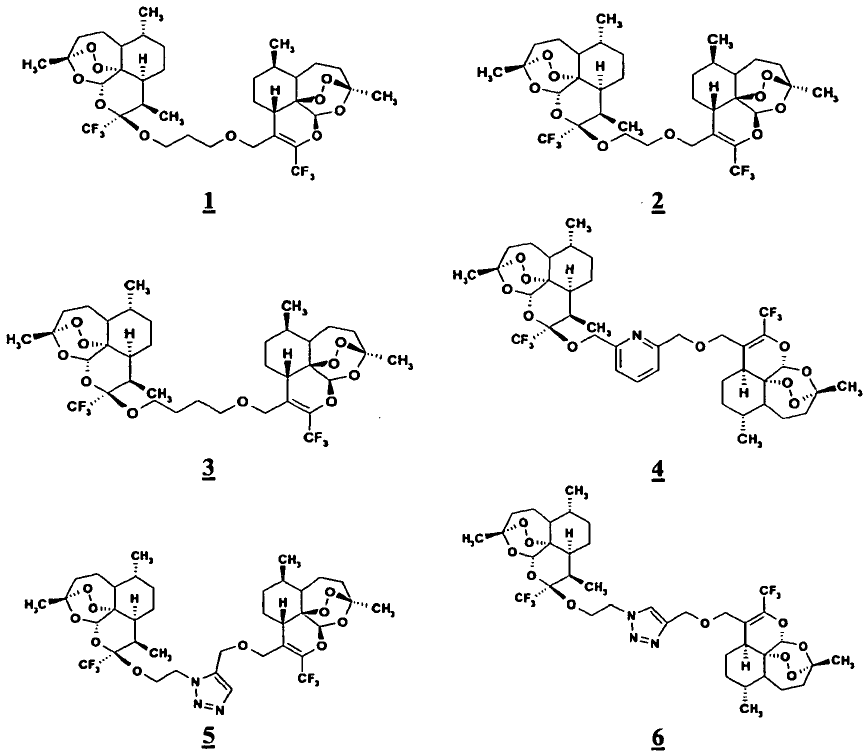

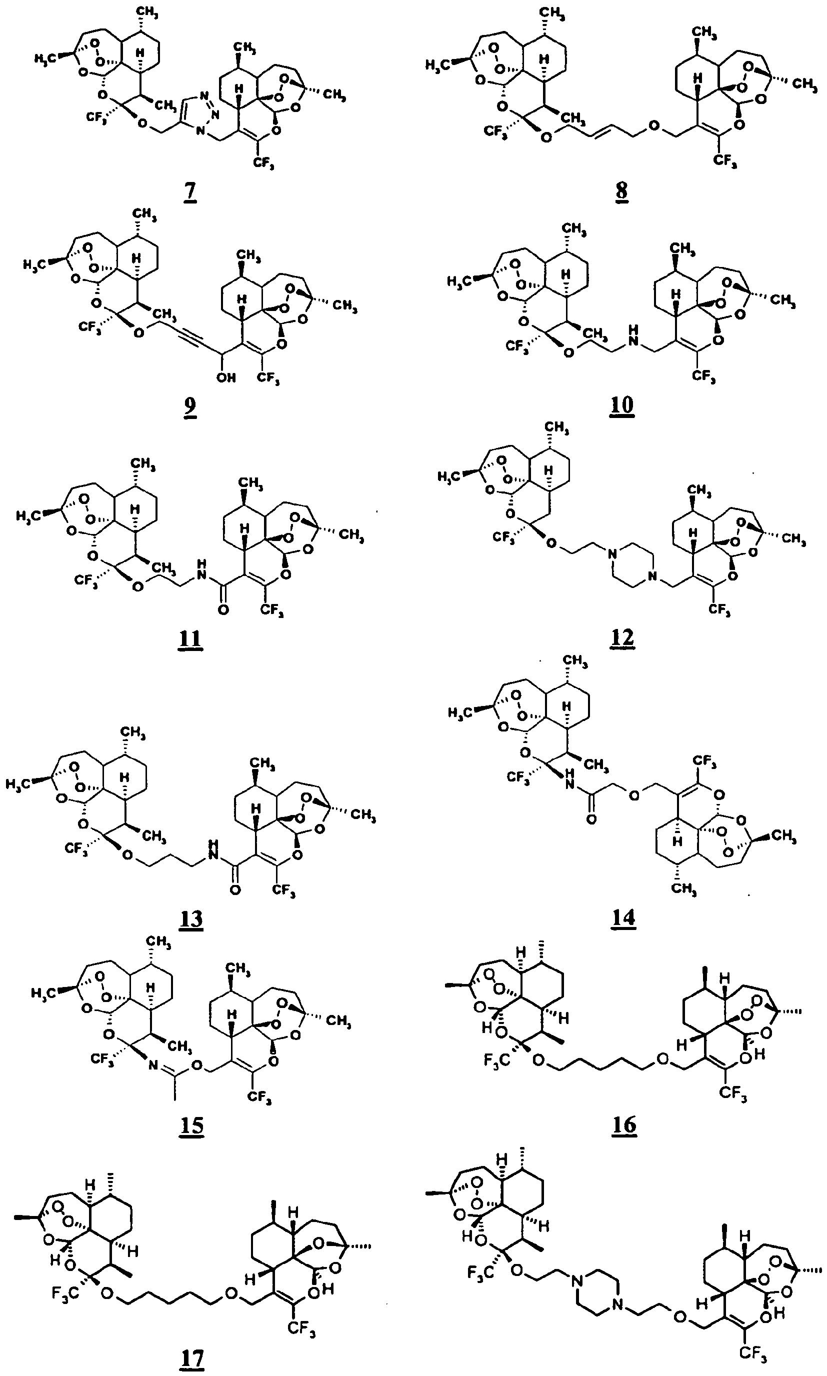

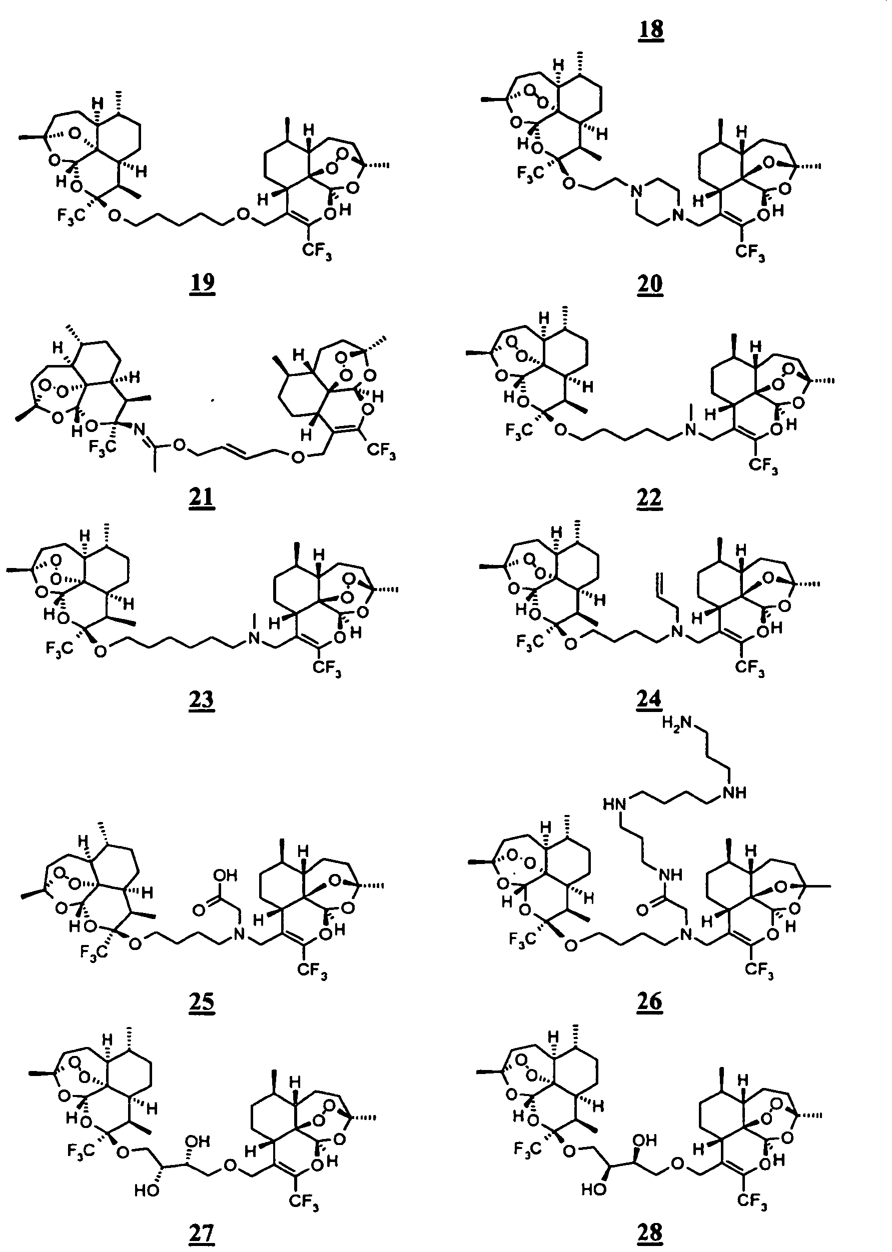

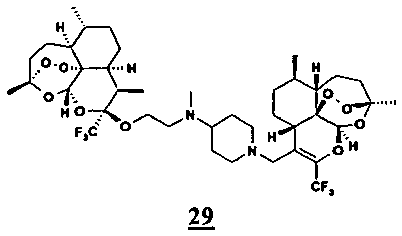

6. Dérivé dimérique selon l'une quelconque des revendications 1 à 5 pour son utilisation en tant que médicament, notamment destiné au traitement du cancer.

7. Composition pharmaceutique comprenant au moins un dérivé dimérique selon l'une quelconque des revendications 1 à 5 et au moins un véhicule pharmaceutiquement acceptable.

8. Composition pharmaceutique selon la revendication 7, **caractérisé en ce qu'**elle comprend en outre au moins un autre principe actif, avantageusement choisi parmi les agents anticancéreux tels que la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

9. Composition pharmaceutique comprenant :
(i) au moins un composé selon l'une quelconque des revendications 1 à 5, et
(ii) au moins un autre principe actif,
en tant que produits de combinaison pour une utilisation simultanée, séparée ou étalée dans le temps.

10. Composition selon la revendication 9, **caractérisée en ce que** le(s) principe(s) actif(s) est(sont) choisi(s) parmi les agents anticancéreux tels que la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide ou le lanréotide.

11. Composition selon l'une quelconque des revendications 7 à 10, pour son utilisation en tant que médicament destiné au traitement du cancer.

12. Procédé de préparation d'un composé de formule (I) selon l'une quelconque des revendications 1 à 5, **caractérisé en ce que** le composé de formule (I) est obtenu par couplage d'un composé de formule (II) suivante : 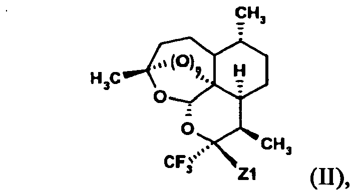 pour laquelle a est tel que défini à la revendication 1 et Z1 représente un atome d'halogène, de préférence un atome de brome, ou un groupement -OH, -NH₂, ou -NHR1, R1 étant tel que défini à la revendication 1,
avec un composé de formule (III) suivante : 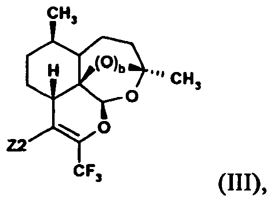 pour laquelle b est tel que défini à la revendication 1 et Z2 représente un groupement - CH₂OH, -CHO, -COOH, -CH₂N₃, -CH₂NH₂ ou -CH₂Hal, où Hal représente un atome d'halogène, de préférence un atome de brome.

13. Procédé selon la revendication 12, **caractérisé en ce qu'**un composé de formule (I) pour lequel B = -CH₂Y- -ou -C(=O)Y-, avec Y tel que défini à la revendication 1, est préparé par couplage entre les composés (II) et (III) et un composé de formule (IV) suivante :
Z3-X-Z4 (IV),
avec :
X tel que défini à la revendication 1,
Z3 représentant un atome d'halogène, tel qu'un atome de brome ou de chlore, ou un groupement -OH, -NHR1, -SH, -N₃ ou -C(=O)R4, R1 étant tel que défini à la revendication 1 et R4 représentant un atome d'hydrogène ou un groupement (C₁-C₆)alkyle, et
Z4 représentant un atome d'halogène, tel qu'un atome de brome ou de chlore, ou un groupement -OH, -NHR1, -SH, -N₃ ou un hétérocycle comportant au moins un groupement NH intracyclique, R1 étant tel que défini à la revendication 1.

14. Procédé selon la revendication 12, **caractérisé en ce qu'**un composé de formule (I) pour lequel B = -CH(OR3)-, avec R3 tel que défini à la revendication 1, est préparé par couplage entre les composés (II) et (III), avec Z2 = -CHO, et un composé de formule (V) suivante :
Z5-X-Z6 (V),
avec :
X tel que défini à la revendication 1,
Z5 représentant un atome d'halogène, tel qu'un atome de brome ou de chlore, ou un groupement -OH, -NHR1, -SH, -N₃ ou -C(=O)R4, R1 étant tel que défini à la revendication 1 et R4 représentant un atome d'hydrogène ou un groupement (C₁-C₆)alkyle, et
Z6 représentant un atome d'hydrogène acide ou un atome d'halogène.

## Patentansprüche

1. Dimeres Derivat von 10-Trifluormethylartemisinin der Formel (I): 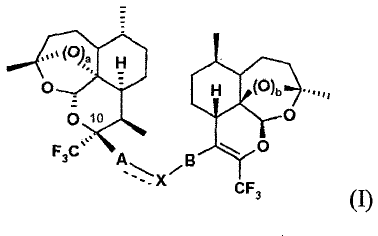 oder ein pharmazeutisch annehmbares Salz desselben,
bei dem:
- a und b unabhängig voneinander 1 oder 2 darstellen, aber nicht gleichzeitig 1 darstellen können,
- A darstellt:
o ein Heteroatom, das aus einem Stickstoff-, Sauerstoff- und Schwefelatom ausgewählt ist, wobei das Stickstoffatom gegebenenfalls durch eine Rest R1 substituiert ist, der ausgewählt ist aus einem Wasserstoffatom, einer (C₁-C₆)-Alkyl-, (C₂-C₆)-Alkenyl-, (C₂-C₆)-Alkinyl-, (C₃-C₈)-Cycloalkyl-, Aryl-(C₁-C₆)-alkyl-, Heteroaryl-(C₁-C₆)-alkyl-, Heterocyclus-(C₁-C₆)-alkyl-, gegebenenfalls substituierten Aryl-, gegebenenfalls substituierten Heteroaryl-, -COR2-, -CO₂R2-, C(O)NR2R2bis-, -SO₂R2-, -CH₂C(O)OR2-und -CH₂C(O)NR2R2bis-Gruppe,
wobei R2 ein Wasserstoffatom oder eine (C₁-C₆)-Alkyl-, (C₂-C₆)-Alkenyl-, (C₂-C₆)-Alkinyl-, gegebenenfalls substituierte Polyamino-, (C₃-C₈)-Cycloalkyl-, Aryl-(C₁-C₆)-alkyl-, Heteroaryl-(C₁-C₆)-alkyl-, gegebenenfalls substituierte Aryl- oder gegebenenfalls substituierte Heteroaryl-Gruppe darstellt und R2bis ein Wasserstoffatom oder eine (C₁-C₆)-Alkylgruppe darstellt, oder
o einen gesättigten Heterocyclus, der ein oder mehrere Heteroatome umfasst, die aus Sauerstoff-, Schwefel-, Stickstoffatomen, darunter mindestens ein an den Kohlenstoff 10 gebundenes Stickstoffatom, ausgewählt sind,
- 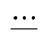 eine Einfachbindung darstellt, wenn A ein Sauerstoff- oder Schwefelatom oder einen Heterocyclus darstellt, oder eine Einfachbindung oder eine Doppelbindung darstellt, wenn A ein Stickstoffatom darstellt, wobei das Stickstoffatom durch einen wie vorstehend definierten Rest R1 substituiert ist, wenn 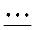 eine Einfachbindung darstellt,
- B eine Gruppe -CH₂-Y-, -C(=O)-Y- oder -CH(OR3)- darstellt,
wobei Y O, S, N-R1 oder einen Heterocyclus darstellt, wobei R1 wie vorstehend definiert ist,
und
R3 ein Wasserstoffatom oder eine (C₁-C₆)-Alkyl- oder Arylgruppe darstellt, und
- X darstellt:
● wenn 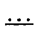 eine Doppelbindung darstellt:
eine Gruppe =C(X1)-(O-X2)_{c}-, wobei X1 und X2 unabhängig voneinander eine (C₁-C₆)-Alkyl- oder (C₂-C₆)-Alkenylgruppe darstellen und c 0 oder 1 darstellt, oder
● wenn 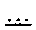 eine Einfachbindung darstellt:
o eine gegebenenfalls mit einer oder mehreren OH-Gruppen substituierte (C₁-C₆)-Alkyl-; (C₂-C₆)-Alkenyl-; (C₂-C₆)-Alkinyl-; [(C₁-C₆)Alkyl]ₙ-(C₃-C₈)-cycloalkyl-[(C₁-C₆)-alkyl]ₚ-; [(C₁-C₆)-Alkyl]ₙ-heterocyclus-[(C₁-C₆)-alkyl]p-; [(C₁-C₆)-Alkyl]ₙ-aryl-[(C₁-C₆)-alkyl]ₚ-; [(C₁-C₆)-Alkyl]ₙ-heteroaryl-[(C₁-C₆)-alkyl]ₚ-Gruppe, wobei n und p unabhängig voneinander 0 oder 1 darstellen,
o eine -CO-(CH₂)_{q}- oder -CO-(CH₂)_{q}-CO-Gruppe, bei der q eine ganze Zahl gleich 1, 2, 3 oder 4 darstellt, oder
o eine -COᵣ-(CH₂)ₛ-Z-(CH₂)ₜ-COᵤ-Gruppe, bei der r und u unabhängig voneinander eine ganze Zahl gleich 0 oder 1 darstellen,
s und t unabhängig voneinander eine ganze Zahl gleich 0, 1, 2, 3 oder 4 darstellen und
Z eine -S-, -S-S-, -SO-, -SO₂-, -Se-Se-, -O-P(O)(OR3)-O-, -NR1-, (C₃-C₈)-Cycloalkyl-, Aryl- oder Heteroarylgruppe darstellt, wobei R1 und
R3 wie vorstehend definiert sind.

2. Dimeres Derivat nach Anspruch 1, **dadurch gekennzeichnet, dass** A ein Sauerstoff-oder Stickstoffatom darstellt.

3. Dimeres Derivat nach irgendeinem der Ansprüche 1 und 2, **dadurch gekennzeichnet, dass** B eine -CH₂O-, -CH₂NR1-, -C(=O)NR1-, -CH(OR3)- und -CH₂-(Heterocyclus)-Gruppe darstellt, wobei R1 und R3 wie in Anspruch 1 definiert sind.

4. Dimeres Derivat nach irgendeinem der Ansprüche 1 bis 3, **dadurch gekennzeichnet, dass** 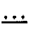 eine Einfachbindung darstellt und X eine (C₁-C₆)-Alkyl-, (C₂-C₆)-Alkenyl-, (C₂-C₆)-Alkinyl-, (C₁₋C₆)-Alkyl-heteroaryl-(C₁-C₆)-alkyl-, -(CH₂)_{q}-NR1- oder -CO-(CH₂)_{q}- Gruppe darstellt, wobei q wie in Anspruch 1 definiert ist.

5. Dimeres Derivat nach irgendeinem der Ansprüche 1 bis 4, **dadurch gekennzeichnet, dass** es aus den folgenden Verbindungen ausgewählt ist:

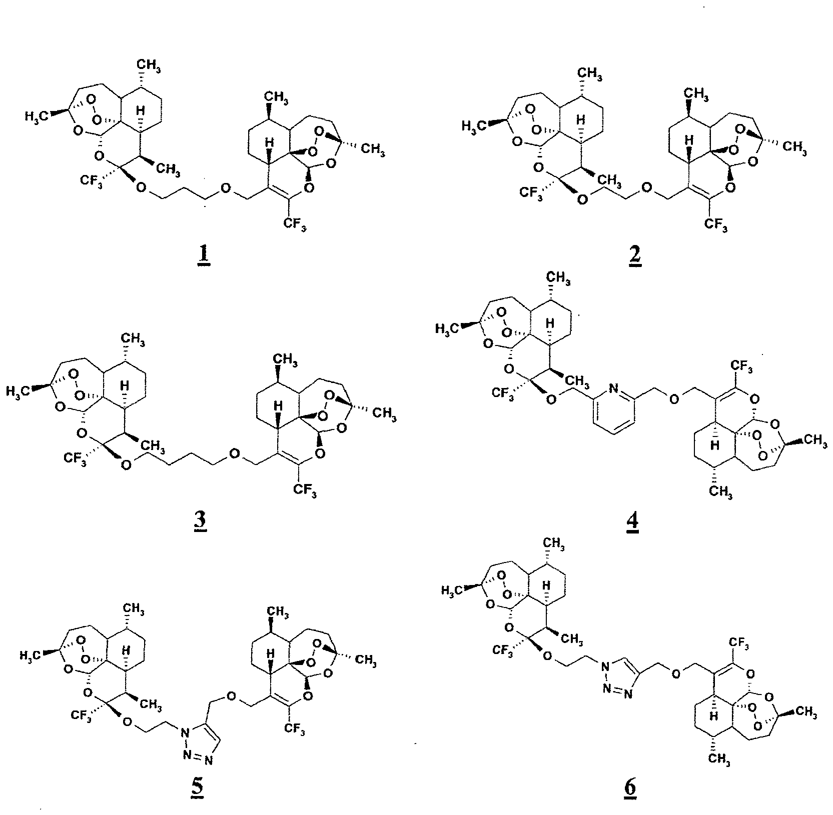

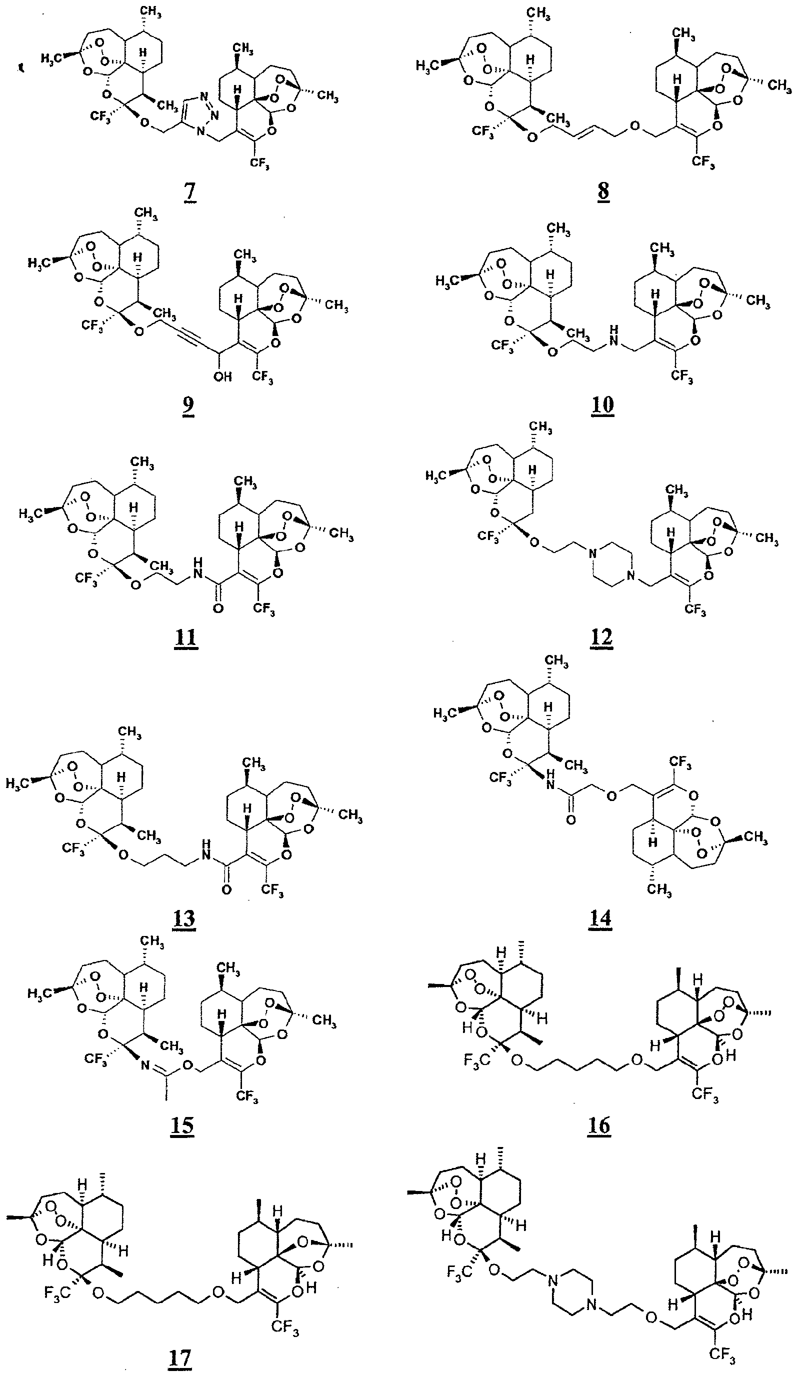

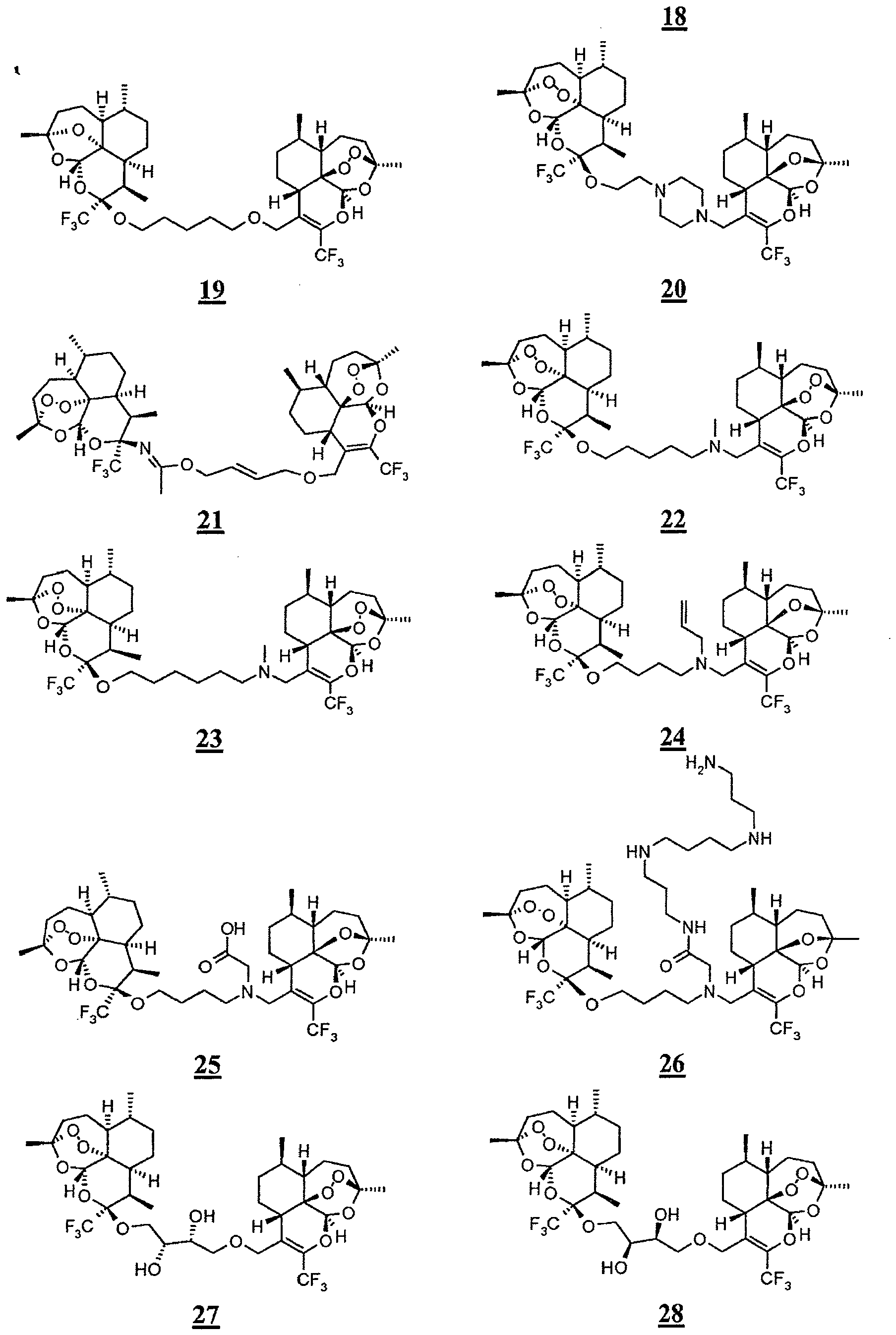

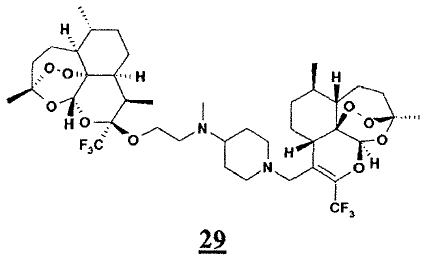

6. Dimeres Derivat nach irgendeinem der Ansprüche 1 bis 5 zur Verwendung als Medikament, das insbesondere für die Behandlung von Krebs bestimmt ist.

7. Pharmazeutische Zusammensetzung, umfassend mindestens ein dimeres Derivat nach irgendeinem der Ansprüche 1 bis 5 und mindestens ein pharmazeutisch annehmbares Vehikel.

8. Pharmazeutische Zusammensetzung nach Anspruch 7, **dadurch gekennzeichnet, dass** sie mindestens einen anderen Wirkstoff umfasst, der vorteilhaft aus Antikrebsmitteln ausgewählt ist, wie 6-Mercaptopurin, Fludarabin, Cladribin, Pentostatin, Cytarabin, 5-Fluoruracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid und Lanreotid.

9. Pharmazeutische Zusammensetzung, umfassend:
(i) mindestens eine Verbindung nach irgendeinem der Ansprüche 1 bis 5, und
(ii) mindestens einen anderen Wirkstoff
als Kombinationsprodukte für eine gleichzeitige, getrennte oder zeitlich gestaffelte Verwendung.

10. Zusammensetzung nach Anspruch 9, **dadurch gekennzeichnet, dass** der oder Wirkstoff(e) aus Antikrebsmitteln ausgewählt ist/sind, wie 6-Mercaptopurin, Fludarabin, Cladribin, Pentostatin, Cytarabin, 5-Fluoruracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid oder Lanreotid.

11. Zusammensetzung nach irgendeinem der Ansprüche 7 bis 10 für die Verwendung als Medikament, das zur Behandlung von Krebs bestimmt ist.

12. Verfahren zur Herstellung einer Verbindung der Formel (I) nach irgendeinem der Ansprüche 1 bis 5, **dadurch gekennzeichnet, dass** die Verbindung der Formel (I) durch Kuppeln einer Verbindung der folgenden Formeln (II), 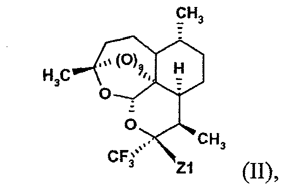 bei der a wie im Anspruch 1 definiert ist und Z1 ein Halogenatom, vorzugsweise ein Bromatom, oder eine OH-, -NH₂- oder -NHR1-Gruppe darstellt, wobei R1 wie in Anspruch 1 definiert ist,
mit einer Verbindung der folgenden Formel (III): 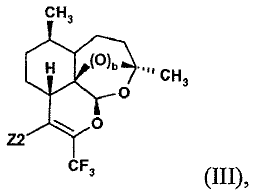 bei der b wie in Anspruch 1 definiert ist und Z2 eine -CH₂OH-, -CHO-,-COOH-, -CH₂N₃-, -CH₂NH₂- oder -CH₂Hal-Gruppe darstellt, worin Hal ein Halogenatom, vorzugsweise ein Bromatom darstellt,
erhalten wird.

13. Verfahren nach Anspruch 12, **dadurch gekennzeichnet, dass** eine Verbindung der Formel (I), bei der B = -CH₂Y- oder -C(=O)Y- ist, wobei Y wie in Anspruch 1 definiert ist, durch Kuppeln zwischen den Verbindungen (II) und (III) und einer Verbindung der folgenden Formel (IV) hergestellt wird:
Z3-X-Z4 (IV),
wobei:
X wie in Anspruch 1 definiert ist,
Z3 ein Halogenatom, wie ein Brom- oder Chlor-Atom, oder eine -OH-, -NHR1-, -SH-, -N₃- oder -C(=O)R4-Gruppe darstellt, wobei R1 wie in Anspruch 1 definiert ist und R4 ein Wasserstoffatom oder eine (C₁-C₆)-Alkylgruppe darstellt und
Z4 ein Halogenatom, wie ein Brom- oder Chloratom, oder eine -OH-, -NHR1-, -SH-, -N₃-Gruppe oder einen Heterocyclus darstellt, der mindestens eine intracyclische NH-Gruppe umfasst, wobei R1 wie in Anspruch 1 definiert ist.

14. Verfahren nach Anspruch 12, **dadurch gekennzeichnet, dass** eine Verbindung der Formel (I), bei der B = -CH(OR3)- ist, wobei R3 wie in Anspruch 1 definiert ist, durch Kuppeln zwischen den Verbindungen (II) und (III), wobei Z2 = -CHO, und einer Verbindung der folgenden Formel (V) hergestellt wird:
Z5-X-Z6 (V),
wobei:
X wie in Anspruch 1 definiert ist,
Z5 ein Halogenatom, wie ein Chlor- oder Bromatom, oder eine -OH-, -NHR1-, -SH-, -N₃-oder -C(=O)R4-Gruppe darstellt, wobei R1 wie in Anspruch 1 definiert ist und R4 ein Wasserstoffatom oder eine (C₁-C₆)-Alkylgruppe darstellt und
Z6 ein saures Wasserstoffatom oder ein Halogenatom darstellt.

## Claims

1. A dimeric derivative of 10-trifluoromethylated artemisinin of formula (I): 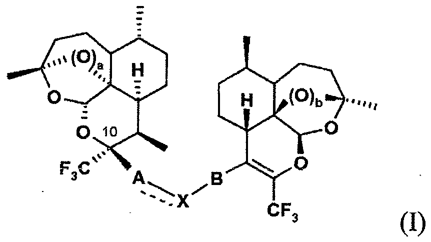 or a pharmaceutically acceptable salt thereof, wherein:
- a and b represent, independently of each other, 1 or 2 but cannot represent 1 at the same time,
- A represents:
o a heteroatom selected from an atom of nitrogen, oxygen and sulphur,
the nitrogen atom being optionally substituted by a radical R1 selected from a hydrogen atom and the following groups: (C₁-C₆)alkyl, (C₂-C₆)alkenyl, (C₂-C₆)alkynyl, (C₃-C₈)cycloalkyl, aryl-(C₁-C₆)alkyl, heteroaryl-(C₁-C₆)alkyl, heterocycle-(C₁-C₆)alkyl, optionally substituted aryl, optionally substituted heteroaryl, -COR2, -CO₂R2, -C(O)NR2R2a, -SO₂R2, -CH₂C(O)OR2 and -CH₂C(O)NR2R2a, with R2 representing a hydrogen atom or one of the following groups: (C₁-C₆)alkyl, (C₂-C₆)alkenyl, (C₂-C₆)alkynyl, optionally substituted polyamine, (C₃-C₈)cycloalkyl, aryl-(C₁-C₆)alkyl, heteroaryl-(C₁-C₆)alkyl, optionally substituted aryl or optionally substituted heteroaryl, and R2a representing a hydrogen atom or a (C₁-C₆)alkyl group, or
o a saturated heterocycle comprising one or more heteroatoms selected from atoms of oxygen, sulphur and nitrogen, of which at least one nitrogen atom is linked to carbon 10,
- 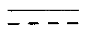 represents a single bond when A represents an atom of oxygen or sulphur or a heterocycle, or represents a single bond or a double bond when A represents a nitrogen atom, the aforesaid nitrogen atom being substituted by a radical R1 as defined above when 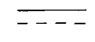 represents a single bond,
- B represents a -CH₂-Y-, -C(=O)-Y- or -CH(OR3)-group,
with Y representing O, S, N-R1 or a heterocycle, with R1 as defined above, and
R3 representing a hydrogen atom or a (C₁-C₆)alkyl or aryl group, and
- X represents:
o when 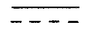 represents a double bond:
a =C(X1)-(O-X2)_{c}- group with X1 and X2 representing, independently of each other,
a (C₁-C₆)alkyl or (C₂-C₆)alkenyl group and c representing 0 or 1, or
o when 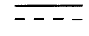 represents a single bond:
■ one of the following groups: (C₁-C₆)alkyl optionally substituted by one or more OH groups; (C₂-C₆)alkenyl; (C₂-C₆)alkynyl; [(C₁-C₆)alkyl]ₙ-(C₃-C₈) cycloalkyl- [(C₁-C₆)alkyl]ₚ; [(C₁-C₆)alkyl]ₙ-heterocycle[(C₁-C₆)alkyl]ₚ;
[(C₁-C₆)alkyl]ₙ-aryl]ₙ-aryl-[(C₁-C₆)alkyl]ₚ;
[(C₁-C₆)alkyl]ₙ-heteroaryl-[(C₁-C₆)alkyl]ₚ; with n and p representing, independently of each other, 0 or 1,
■ a -CO-(CH₂)_{q}- or -CO-(CH₂)_{q}-CO- group for which q represents an integer equal to 1, 2, 3 or 4, or
■ a -COᵣ-(CH₂)ₛ-Z-(CH₂)ₜ-COᵤ- group for which r and u represent, independently of each other, an integer equal to 0 or 1,
s and t represent, independently of each other, an integer equal to 0, 1, 2, 3 or 4, and
Z represents an -S-, -S-S-, -SO-, -SO₂-, -Se-Se-, -O-P(O)(OR3)-O-, -NR1-, (C₃-C₈)-cycloalkyl, aryl or heteroaryl group, with R1 and R3 as defined above.

2. The dimeric derivative of claim 1, wherein A represents an oxygen or nitrogen atom.

3. The dimeric derivative of either of claims 1 and 2, wherein B represents a -CH₂O-, -CH₂NR1-, -C(=O)NR1-, -CH (OR3) - or -CH₂-(heterocycle)- group, with R1 and R3 as defined in claim 1.

4. The dimeric derivative of any of claims 1 to 3, wherein 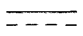 represents a single bond and X represents a (C₁-C₆)alkyl, (C₂-C₆)alkenyl, (C₂-C₆)alkynyl, (C₁-C₆)alkyl-heteroaryl-(C₁-C₆)alkyl, -(CH₂)_{q}-NR1- or -CO-(CH₂)_{q}- group, with q as defined in claim 1.

5. The dimeric derivative of any of claims 1 to 4, wherein it is selected from the following compounds:

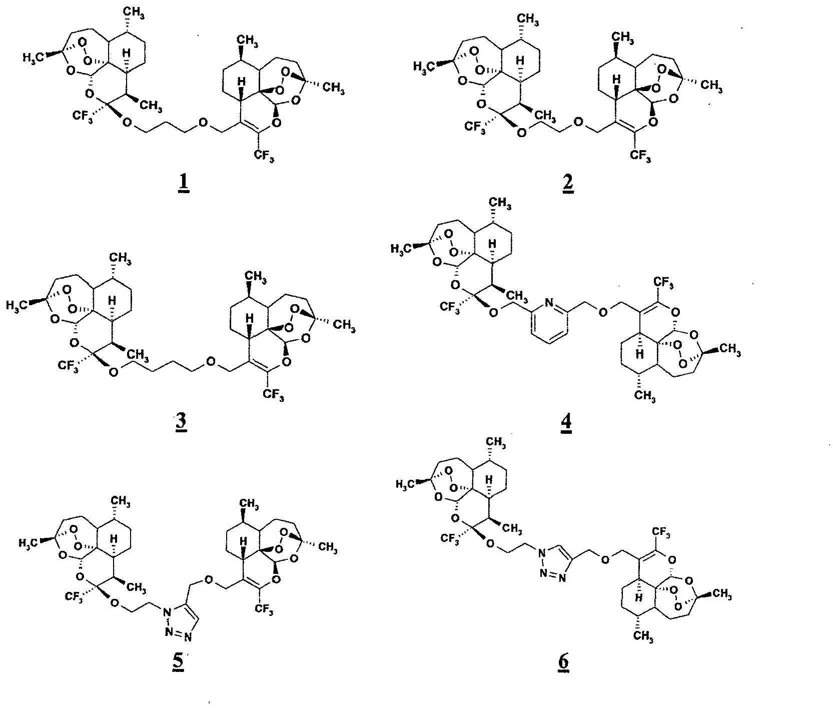

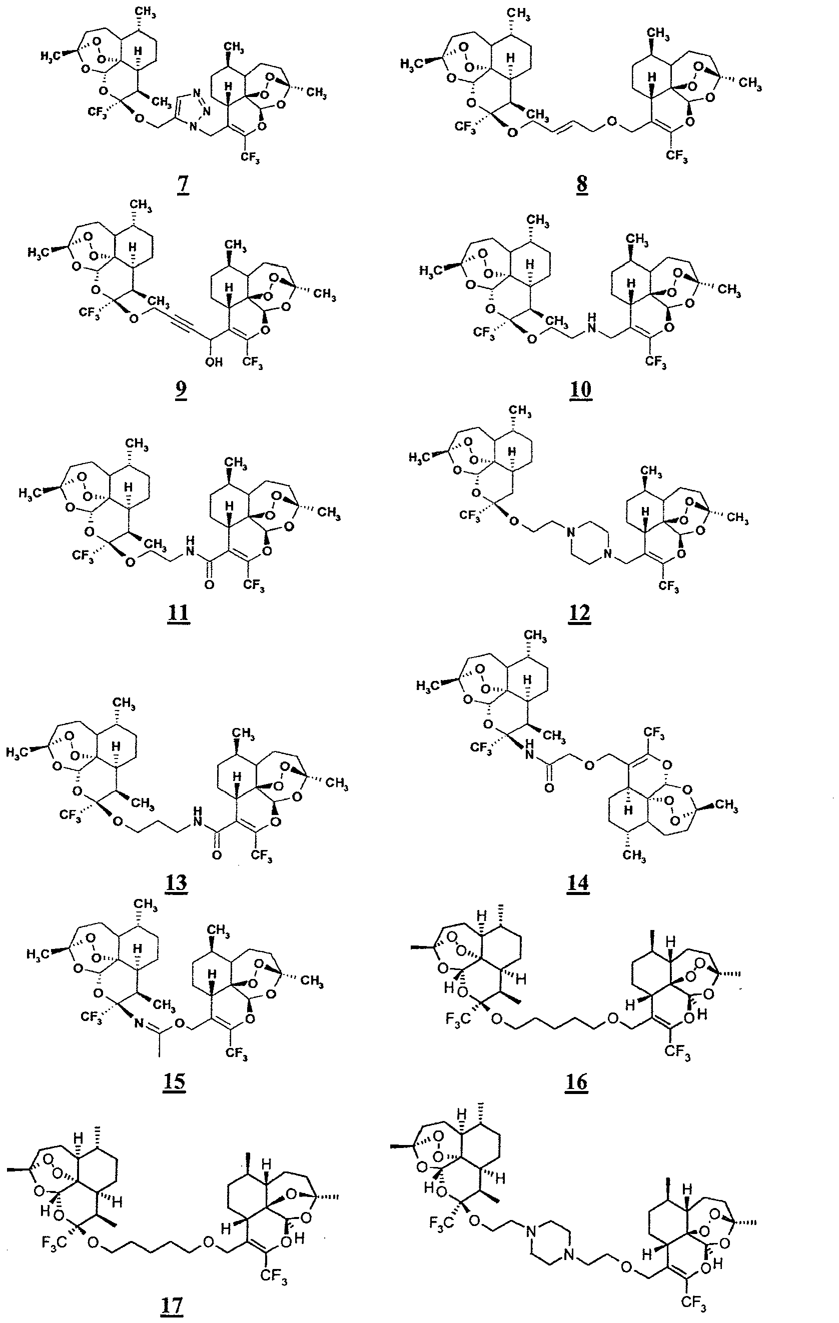

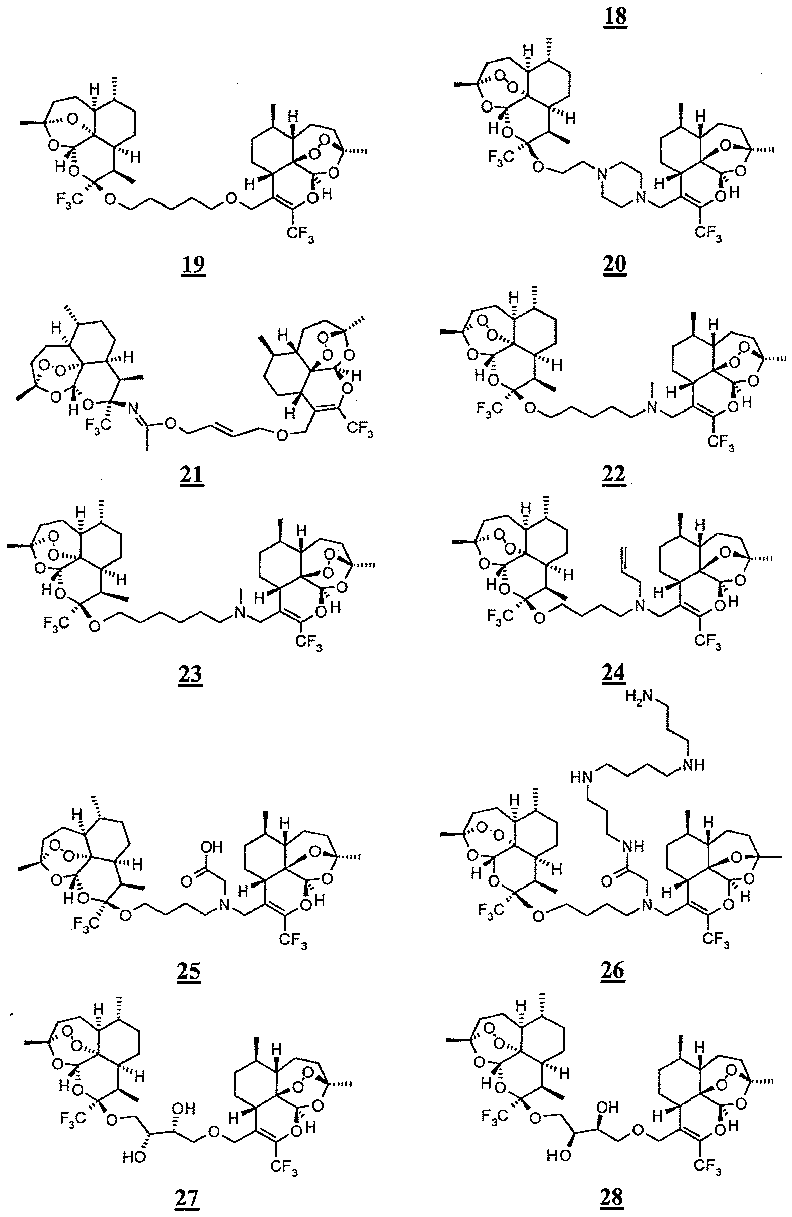

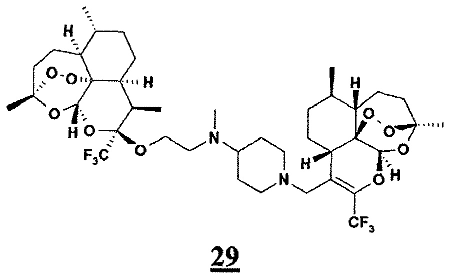

6. A dimeric derivative of any of claims 1 to 5 for the use thereof as a drug, notably for treating cancer.

7. A pharmaceutical composition comprising at least one dimeric derivative of any of claims 1 to 5 and at least one pharmaceutically acceptable carrier.

8. The pharmaceutical composition of claim 7, wherein it further comprises at least one other active ingredient, advantageously selected from anticancer agents such as 6-mercaptopurine, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrozole, letrozole, tamoxifen, octreotide and lanreotide.

9. A pharmaceutical composition comprising:
(i) at least one compound of any of claims 1 to 5, and
(ii) at least one other active ingredient,
as combination products for a simultaneous, separated or sequential use.

10. The composition of claim 9, wherein the active ingredient(s) is/are selected from anticancer agents such as 6-mercaptopurine, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrozole, letrozole, tamoxifen, octreotide or lanreotide.

11. The composition of any of claims 7 to 10, for the use thereof as a drug for treating cancer.

12. A method for preparing a compound of formula (I) of any of claims 1 to 5, wherein the compound of formula (I) is obtained by coupling a compound of following formula (II): 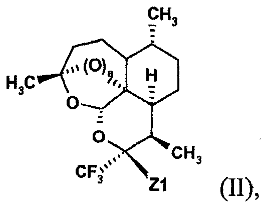 wherein a is as defined in claim 1 and Z1 represents a halogen atom, preferably a bromine atom, or an -OH, -NH₂ or -NHR1 group, with R1 as defined in claim 1, with a compound of following formula (III): 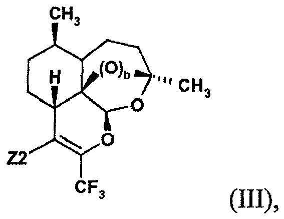 wherein b is as defined in claim 1 and Z2 represents a -CH₂OH, -CHO, -COOH, -CH₂N₃, -CH₂NH₂ or -CH₂Hal group, where Hal represents a halogen atom, preferably a bromine atom.

13. The method of claim 12, wherein a compound of formula (I) in which B=-CH₂Y- or -C(=O)Y-, with Y as defined in claim 1, is prepared by coupling between compounds (II) and (III) and a compound of following formula (IV):
Z3-X-Z4 (IV),
with:
X as defined in claim 1,
Z3 representing a halogen atom, such as a bromine or
chlorine atom, or an -OH, -NHR1, -SH, -N₃ or -C(=O)R4 group, with R1 as defined in claim 1 and R4 representing a hydrogen atom or a (C₁-C₆)alkyl group,
and
Z4 representing a halogen atom, such as a bromine or chlorine atom, or an -OH, -NHR1, -SH or -N₃ group or a heterocycle comprising at least one intracyclic NH group, with R1 as defined in claim 1.

14. The method of claim 12, wherein a compound of formula (I) in which B=-CH(OR3)-, with R3 as defined in claim 1, is prepared by coupling between compounds (II) and (III), with Z2=-CHO, and a compound of following formula (V):
Z5-X-Z6 (V),
with:
X as defined in claim 1,
Z5 representing a halogen atom, such as a bromine or
chlorine atom, or an -OH, -NHR1, -SH, -N₃ or -C(=O)R4 group, with R1 as defined in claim 1 and R4 representing a hydrogen atom or a -(C₁-C₆)alkyl group,
and
Z6 representing an acid hydrogen atom or a halogen atom.
